# EUROPEAN PATENT SPECIFICATION

(11) **EP 0 915 989 B1**
(45) Date of publication and mention of the grant of the patent: **02.06.2010**
(21) Application number: 97934976.8
(22) Date of filing: 16.07.1997
(51) Int. Cl.: C12Q 1/48, C12Q 1/68, C07K 19/00, C12N 15/62, C12N 15/11, C12N 1/21, C12N 15/10, C12N 15/09

(54) **ASSAYS FOR PROTEIN KINASES USING FLUORESCENT PROTEIN SUBSTRATES**
TESTS AUF PROTEINKINASEN MIT FLUORESZIERENDEN PROTEINSUBSTRATEN
ESSAIS DES PROTEINES KINASES FAISANT APPEL A DES SUBSTRATS PROTEIQUES FLUORESCENTS

(30) Priority: 16.07.1996 US 679865; 16.07.1996 US 680876; 16.07.1996 US 680877
(43) Date of publication of application: 19.05.1999
(73) Proprietor: The Regents of The University of California, Oakland, California 94612-3550 (US)
(72) Inventor: TSIEN, Roger, Y., La Jolla, CA 92037 (US); CUBITT, Andrew, B., San Diego, CA 92131 (US)
(74) Representative: Vossius & Partner
(86) International application number: PCT/US1997/012410
(87) International publication number: WO 1998/002571

(56) References cited:
- WO-A-91/01305
- WO-A-96/23898
- US-A- 5 432 018
- US-A- 5 532 167
- MCILROY B.K. ET AL.: "A CONTINUOUS FLUORESCENCE ASSAY FOR PROTEIN KINASE C" ANALYTICAL BIOCHEMISTRY, ACADEMIC PRESS, SAN DIEGO, CA, US, vol. 195, no. 1, 1991, pages 148-152, XP002254376 ISSN: 0003-2697
- CORMACK B.P. ET AL.: "FACS-OPTIMIZED MUTANTS OF THE GREEN FLUORESCENT PROTEIN (GFP)" GENE: AN INTERNATIONAL JOURNAL ON GENES AND GENOMES, ELSEVIER SCIENCE PUBLISHERS, BARKING, GB, vol. 173, no. 1, 1 July 1996 (1996-07-01), pages 33-38, XP002037756 ISSN: 0378-1119
- EHRIG T. ET AL.: "GREEN-FLUORESCENT PROTEIN MUTANTS WITH ALTERED FLUORENCE EXCITATION SPECTRA" FEBS LETTERS, ELSEVIER, AMSTERDAM, NL, vol. 367, 19 June 1995 (1995-06-19), pages 163-166, XP000579119 ISSN: 0014-5793
- HEIM R. ET AL.: "ENGINEERING GREEN FLUORESCENT PROTEIN FOR IMPROVED BRIGHTNESS, LONGER WAVELENGTHS AND FLUROESCENCE RESONANCE ENERGY TRANSFER" CURRENT BIOLOGY, CURRENT SCIENCE,, GB, vol. 6, no. 2, 1 February 1996 (1996-02-01), pages 178-182, XP000676582 ISSN: 0960-9822
- CUBITT A.B. ET AL.: "Understanding, improving and using green fluorescent proteins" TIBS TRENDS IN BIOCHEMICAL SCIENCES, ELSEVIER PUBLICATION, CAMBRIDGE, EN, vol. 20, no. 11, November 1995 (1995-11), pages 448-455, XP004222335 ISSN: 0968-0004
- PRASHER D.C. ET AL.: "PRIMARY STRUCTURE OF THE AEQUOREA VICTORIA GREEN-FLUORESCENT PROTEIN" GENE, ELSEVIER, AMSTERDAM, NL, vol. 111, 1992, pages 229-233, XP001018985 ISSN: 0378-1119
- KEMP B.E. ET AL.: "PROTEIN KINASE RECOGNITION SEQUENCE MOTIFS" TRENDS IN BIOCHEMICAL SCIENCES, vol. 15, no. 9, 1990, pages 342-346, XP001207074 ISSN: 0968-0004
- SAWIN K.E. ET AL.: "Identification of fission yeast nuclear markers using random polypeptide fusions with green fluorescent protein" PROCEEDINGS OF THE NATIONAL ACADEMY OF SCIENCES OF USA, WASHINGTON, US, vol. 94, no. 26, 24 December 1996 (1996-12-24), pages 15146-15151, XP002121720 ISSN: 0027-8424
- WRIGHT D.E. ET AL.: 'Fluorometric Assay for Adenosine 3',5'-Cyclic Monophosphate-Dependent Protein Kinase and Phosphoprotein Phosphatase Activities' PROC. NATL. ACAD. SCI. U.S.A. vol. 78, no. 10, 1981, pages 6048 - 6050, XP001029505
- MALENCIK D.A. ET AL.: 'Characterization of a Fluorescent Substrate for the Adenosine 3',5'-Cyclic Monophosphate-Dependent Protein Kinase' ANAL. BIOCHEM. vol. 132, 1983, pages 34 - 40, XP002991977
- ZHAO Z.: 'Characterization of a New Substrate for Protein Kinase C: Assay by Continuous Fluorometric Monitoring and High Performance Liquid Chromatography' BIOCHEM. BIOPHYS. RES. COMMUN. vol. 176, no. 3, 15 May 1991, pages 1454 - 1461, XP002991978
- SALA-NEWBY G.B. ET AL.: 'Engineering a Bioluminescent Indicator for Cyclic AMP-Dependent Protein Kinase.' BIOCHEM. J. vol. 279, 1991, pages 727 - 732, XP002945392

## Description

### BACKGROUND OF THE INVENTION

This invention relates to the field of enzymatic assays and, in particular, assays for protein kinase activity involving modified fluorescent proteins.

Protein phosphorylation is one of the most important general mechanisms of cellular regulation. Protein phosphorylation commonly occurs on three major amino acids, tyrosine, serine or threonine, and changes in the phosphorylation state of these amino acids within proteins can regulate many aspects of cellular metabolism, regulation, growth and differentiation. Changes in the phosphorylation state of proteins, mediated through phosphorylation by kinases, or dephosphorylation by phosphatases, is a common mechanism through which cell surface signaling pathways transmit and integrate information into the nucleus. Given their key role in cellular regulation, it is not surprising that defects in protein kinases and phosphatases have been implicated in many disease states and conditions. For example, the over-expression of cellular tyrosine kinases such as the EGF or PDGF receptors, or the mutation of tyrosine kinases to produce constitutively active forms (oncogenes) occurs in many cancer cells. Drucker et al. (1996) Nature Medicine 2: 561-56. Protein tyrosine kinases are also implicated in inflammatory signals. Defective Thr/Ser kinase genes have been demonstrated to be implicated in several diseases such as myotonic dystrophy as well as cancer, and Alzheimer's disease (Sanpei et al. (1995) Biochem. Biophys. Res. Commun. 212: 341-6; Sperber et al (1995) Neurosci. Lett. 197: 149-153; Grammas et al (1995) Neurobiology of Aging 16: 563-569; Govoni et al. (1996) Ann. N.Y. Acad. Sci. 777: 332-337).

The involvement of protein kinases and phosphatases in disease states makes them attractive targets for the therapeutic intervention of drugs, and in fact many clinically useful drugs act on protein kinases or phosphatases. Examples include cyclosporin A which is a potent immunosuppressant that binds to cyclophilin. This complex binds to the Ca/calmodulin-dependent protein phosphatase type 2B (calcineurin) inhibiting its activity, and hence the activation of T-cells. (Sigal and Dumont (1992), Schreiber and Crabtree (1992)). Inhibitors of protein kinase C are in clinical trails as therapeutic agents for the treatment of cancer. (Clin. Cancer Res. (1995) 1:113-122) as are inhibitors of cyclin dependent kinase. (J. Mol. Med. (1995) 73:(10):509-14.)

The number of known kinases and phosphatases are growing rapidly as the influence of genomic programs to identify the molecular basis for diseases have increased in size and scope. These studies are likely to implicate many more kinase and phosphatase genes in the development and propagation of diseases in the future, thereby making them attractive targets for drug discovery. However current methods of measuring protein phosphorylation have many disadvantages which prevents or limits the ability to rapidly screen using miniaturized automated formats of many thousands of compounds. This is because many current methods rely on the incorporation and measurement of ³²P into the protein substrates of interest. In whole cells this necessitates the use of high levels of radioactivity to efficiently label the cellular ATP pool and to ensure that the target protein is efficiently labeled with radioactivity. After incubation with test drugs, the cells must be lysed and the protein of interest purified to determine its relative degree of phosphorylation. This method requires high numbers of cells, long preincubation times, careful manipulation and washing steps (to avoid artifactual phosphorylation or dephosphorylation), as well as a method of purification of the target protein. Furthermore, final radioactive incorporation into target proteins is usually very low, giving the assay poor sensitivity. Alternative assay methods, for example based on phosphorylation-specific antibodies using ELISA-type approaches, involve the difficulty of producing antibodies that distinguish between phosphorylated and non-phosphorylated proteins, and the requirement for cell lysis, multiple incubation and washing stages which are time consuming, complex to automate and potentially susceptible to artifacts.

Kinase assays based on purified enzymes require large amounts of purified kinases, high levels of radioactivity, and methods of purification of the substrate protein away from incorporated ³²P-labelled ATP. They also suffer from the disadvantage of lacking the physiological context of the cell, preventing a direct assessment of a drugs toxicity and ability to cross the cells plasma membrane.

Fluorescent molecules are attractive as reporter molecules in many assay systems because of their high sensitivity and ease of quantification. Recently, fluorescent proteins have been the focus of much attention because they can be produced *in vivo* by biological systems, and can be used to trace intracellular events without the need to be introduced into the cell through microinjection or permeabilization. The green fluorescent protein of *Aequorea victoria* is particularly interesting as a fluorescent indicator protein. A cDNA for the protein has been cloned. (D.C. Prasher et al., "Primary structure of the Aequorea victoria green-fluorescent proteins," Gene (1992) 111:229-33.) Not only can the primary amino acid sequence of the protein be expressed from the cDNA, but the expressed protein can fluoresce. This indicates that the protein can undergo the cyclization and oxidation believed to be necessary for fluorescence. The fluorescence of green fluorescent protein is generated from residues S65-Y66-G67.

Fluorescent proteins have been used as markers of gene expression, tracers of cell lineage and as fusion tags to monitor protein localization within living cells. (M. Chalfie et al., "Green fluorescent protein as a marker for gene expression, "Science 263:802-805; A.B. Cubitt et al., "Understanding, improving and using green fluorescent proteins," TIBS 20, November 1995, pp. 448-455. U.S. patent 5,491,084, M. Chalfie and D. Prasher. Furthermore, mutant versions of green fluorescent protein have been identified that exhibit altered fluorescence characteristics, including altered excitation and emission maxima, as well as excitation and emission spectra of different shapes. (R. Heim et al., "Wavelength mutations and posttranslational autoxidation of green fluorescent protein," Proc. Natl. Acad. Sci. USA, (1994) 91:12501-04; R. Heim et al., "Improved green fluorescence," Nature (1995) 373:663-665.) These properties add variety and utility to the arsenal of biologically based fluorescent indicators.

Wright D.E. et al., Proc. Natl. Acad. Sci. USA vol. 78, No. 10, pp. 6048-6050 (1981) discloses a fluorometric assay for adenosine 3',5'-cyclic monophosphate-dependent protein kinase and phosphoprotein phosphatase activities. Malencik D.A. et al., Analytical Biochemistry 132, 34-40 (1983) pertains to the characterization of a fluorescent substrate for the adenosine 3',5'-cyclic monophosphate-dependent protein kinase. Zhao Z. et al., Biochem. Biophys. Res. Commun. Vol. 176, No. 3, p. 1454-1461, 1991, relates to the characterization of a new substrate for protein kinase C assay by continuous fluorometric monitoring and high performance liquid chromatography (HPLC). A continuous fluorescence assay for protein kinase C is disclosed in McIlroy B.K. et al., Analytical Biochemistry, vol. 195, p. 148-152, 1991. Cormack B.P. et al., Gene, vol. 173. no. 1, p. 33-38 (1991) discloses FACS-optimized mutants of the green fluorescent protein (GFP).

There is a need for assays of protein phosphorylation that are simple, sensitive, non-invasive, applicable to living cells and tissues and that avoid the use of any radioactivity.

### SUMMARY OF THE INVENTION

When fluorescent proteins are modified to incorporate a phosphorylation site recognized by a protein kinase, the fluorescent proteins not only can become phosphorylated by the protein kinase, but they also can exhibit different fluorescent characteristics in their un-phosphorylated and phosphorylated forms when irradiated with light having a wavelength within their excitation spectrum. This characteristic makes fluorescent protein substrates particularly useful for assaying protein kinase activity in a sample.

This invention provides methods for determining whether a sample contains protein kinase activity. The methods involve contacting the sample with a phosphate donor, usually ATP, and a fluorescent protein substrate of the invention;
the protein substrate comprising a fluorescent protein with at least 85% sequence identity over any 150 contiguous amino acids with the amino acid sequence of SEQ ID NO:2, further containing a phosphorylation site selected from the group consisting of RRFSV at positions 25-29, RRFSA at positions 69-73, KRDSM at positions 214-218, MRKGSCLF at positions 1-8, MRKGSLLF at positions 1-8, MRRESLLF at positions 1-8, MRRDSCLF at positions 1-8, MSRRDSCF at positions 1-8, MSKRRDLS at positions 1-8, MSRRRRSI at position 1-8, MRRRRSII at positions 1-8, MRRRRSIII at positions -1-8, MRRRRSIIIF at positions -2-8, and MRRRRSIIIIF at positions-3-8, wherein the protein substrate exhibits a different fluorescent property in the phosphorylated state than in the un-phosphorylated state;
exciting the fluorescent protein substrate with light of an appropriate wavelength; and measuring the amount of a fluorescent property that differs in the un-phosphorylated state and phosphorylated state. An amount that is consistent with the presence of the fluorescent protein substrate in its phosphorylated state indicates the presence of protein kinase activity, and an amount that is consistent with the presence of the protein substrate in its un-phosphorylated state indicates the absence of protein kinase activity.

One embodiment of the above method is for determining the amount of protein kinase activity in a sample. In this method, measuring the amount of a fluorescent property in the sample comprises measuring the amount at two or more time points after contacting the sample with a phosphate donor and a fluorescent protein substrate of the invention, and determining the quantity of change or rate of change of the measured amount. The quantity or rate of change of the measured amount reflects the amount of protein kinase activity in the sample.

In another aspect, the invention provides methods for determining whether a cell exhibits protein kinase activity. The methods involve the steps of providing a transfected host cell of the invention that produces a fluorescent protein substrate of the invention as defined above, exciting the protein substrate in the cell with light of an appropriate wavelength; and measuring the amount of a fluorescent property that differs in the unphosphorylated and phosphorylated states. An amount that is consistent with the presence of the protein substrate in its phosphorylated state indicates the presence of protein kinase activity, and an amount that is consistent with the presence of the protein substrate in its un-phosphorylated state indicates the absence of protein kinase activity or the presence of phosphatase activity.

In another aspect, the invention provides methods for determining the amount of activity of a protein kinase in one or more cells from an organism. The methods involve providing a transfected host cell comprising a recombinant nucleic acid molecule comprising expression control sequences operatively linked to a nucleic acid sequence coding for the expression of a fluorescent protein substrate of the invention as defined above, the cell expressing the fluorescent protein substrate; exciting the protein substrate in the cell with light; and measuring the amount of a fluorescent property that differs in the unphosphorylated and phosphorylated states at two or more time points after contacting the sample with a phosphate donor and a fluorescent protein substrate, and determining the quantity or rate of change of the measured amount. The quantity or rate of change of the measured amount reflects the amount of protein kinase activity in the sample.

This invention also provides screening methods for determining whether a compound alters the activity of a protein kinase. The methods involve contacting a sample containing a known amount of protein kinase activity with the compound, a phosphate donor for the protein kinase and a fluorescent protein substrate of the invention as defined above; exciting the protein substrate; measuring the amount of protein kinase activity in the sample as a function of the quantity or rate of change of a fluorescent property that differs in the un-phosphorylated and phosphorylated states; and comparing the amount of activity in the sample with a standard activity for the same amount of the protein kinase. A difference between the amount of protein kinase activity in the sample and the standard activity indicates that the compound alters the activity of the protein kinase.

Another aspect of the drug screening methods involve determining whether a compound alters the protein kinase activity in a cell. The methods involve providing first and second transfected host cells exhibiting protein kinase activity and expressing a fluorescent protein substrate of the invention as defined above; contacting the first cell with an amount of the compound; contacting the second cell with a different amount of the compound; exciting the protein substrate in the first and second cells; measuring the amount of protein kinase activity as a function of the quantity of change or rate of change of a fluorescent property that differs in the un-phosphorylated and phosphorylated states in the first and second cells; and comparing the amount in the first and second cells. A difference in the amount indicates that the compound alters protein kinase activity in the cell.

This invention also provides fluorescent protein substrates for a protein kinase, comprising a fluorescent protein with at least 85% sequence identity over any 150 contiguous amino acids of SEQ ID NO:2, further containing a phosphorylation site selected from the group consisting of RRFSV at positions 25-29, RRFSA at positions 69-73, KRDSM at positions 214-218, MRKGSCLF at positions 1-8, MRKGSLLF at positions 1-8, MRRESLLF at positions 1-8, MRRDSCLF at positions 1-8, MSRRDSCF at positions 1-8, MSKRRDLS at positions 1-8, MSRRRRSI at position 1-8, MRRRRSII at positions 1-8, MRRRRSIII at positions -1-8, MRRRRSIIIF at positions -2-8, and MRRRRSIIIIF at positions -3-8.

Fluorescent protein substrates for a protein kinase comprise a fluorescent protein moiety and a phosphorylation site for a protein kinase. The protein substrate exhibits a different fluorescent property in the phosphorylated state than in the unphosphorylated state. In a preferred embodiment, the fluorescent protein is an *Aequorea*-related fluorescent protein. In another embodiment, the phosphorylation site is located within about 5, 10, 15 or 20 amino acids of a terminus, e.g., the amino-terminus, of the fluorescent protein moiety. In another embodiment, the protein substrate comprises the phosphorylation site more than 20 amino acids from a terminal of the fluorescent protein moiety and within the fluorescent protein moiety. The phosphorylation site can be one recognized by, for example, protein kinase A, a cGMP-dependent protein kinase, protein kinase C, Ca²⁺/calmodulin-dependent protein kinase I, Ca²⁺ / calmodulin-dependent protein kinase II or MAP kinase activated protein kinase type 1.

This invention also provides nucleic acid molecules coding for the expression of a fluorescent protein substrate for a protein kinase of the invention. In one aspect, the nucleic acid molecule is a recombinant nucleic acid molecule comprising expression control sequences operatively linked to a nucleic acid sequence coding for the expression of a fluorescent protein substrate for a protein kinase of the invention as defined above. In another aspect, the invention provides transfected host cells transfected with a recombinant nucleic acid molecule comprising expression control sequences operatively linked to a nucleic acid sequence coding for the expression of a fluorescent protein substrate for a protein kinase of the invention as defined above.

Further described are collections of host cells comprising at least 10 different host cell members, each member comprising the above recombinant nucleic acid molecules.

The collections of cells are useful in determining the specificity of cellular kinases, from either diseased or normal tissues. The screening methods involve providing a collection of transfected host cells of the invention; culturing the collection of host cells under conditions for the expression of the fluorescent protein candidate substrate; and determining for each of a plurality of members from the collection whether the member contains a fluorescent protein candidate substrate that exhibits a fluorescent property different than the fluorescent property exhibited by the non-phosphorylated candidate substrate. The presence of fluorescent protein candidate substrate that exhibits a fluorescent property different than the fluorescent property exhibited by the candidate substrate indicates that the candidate substrate possesses a peptide moiety that can be phosphorylated by the kinase present in the host cells.

### BRIEF DESCRIPTION OF THE DRAWINGS

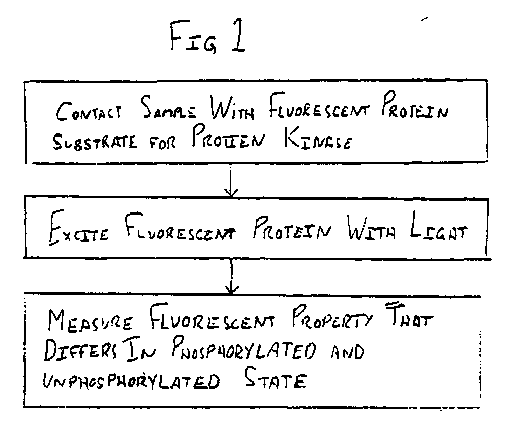

Fig. 1 is a flow chart showing the steps in an assay method for protein kinase activity.
Fig. 2 depicts molecular events in a cell in altering and detecting fluorescent properties of a fluorescent protein substrate for a protein kinase.
Fig. 3 depicts the nucleotide sequence (SEQ ID NO:1) and deduced amino acid sequence (SEQ ID NO:2) of a wild-type *Aequorea* green fluorescent protein.
Fig. 4 provides a list of the positions and amino acid changes made for phosphorylation mutants made more than fifteen amino acids in the primary sequence from the N-terminus, as compared to Fig. 3. Amino acids underlined represent the phosphorylation motif, amino acids in brackets represent wild type sequence at those positions.
Fig. 5 depicts plasmid pRSET containing a region encoding GFP that is fused in frame with nucleotides encoding an N-terminal polyhistidine tag.
Figs. 6A-6E show the fluorescence excitation spectra before and after phosphorylation of N-terminal phosphorylation mutants by protein kinase A using standard phosphorylation conditions. 6A: 1MSRRRRSI (SEQ ID NO:31). 6B: 1MRRRRSII (SEQ ID NO:32). 6C: -1MRRRRSIII (SEQ ID NO:33). 6D: -2MRRRRSIIIF (SEQ ID NO:34). 6E: -3MRRRRSIIIIF (SEQ ID NO:35). In all cases the spectrum after phosphorylation has higher amplitude than the spectrum before phosphorylation.
Fig. 7 depicts an expression vector having expression control sequences operably linked to sequences coding for the expression of protein kinase A catalytic subunit (PKA cat) upstream from sequences coding for the expression of a fluorescent protein substrate.
Fig. 8 depicts the fluorescence excitation spectrum of 1MRRRRSII (SEQ ID NO:33): S65A, N149K, V163A and I167T before and after phosphorylation by protein kinase A using standard phosphorylation conditions. The spectrum after phosphorylation has higher amplitude than the spectrum before phosphorylation.

### DETAILED DESCRIPTION OF THE INVENTION

### 1. METHODS FOR ASSAYING SAMPLES FOR PROTEIN KINASES

Protein kinases add a phosphate residue to the phosphorylation site of a protein, generally through the hydrolysis of ATP to ADP. Fluorescent protein substrates for protein kinases are useful in assays to determine the amount of protein kinase activity in a sample without the need for radioactivity. The assays of this invention take advantage of the fact that phosphorylation of the protein substrate results in a change in a fluorescent property of the fluorescent protein. Methods for determining whether a sample has kinase activity involve contacting the sample with a fluorescent protein substrate having a phosphorylation site recognized by the protein kinase to be assayed and with a phosphate donor under selected test conditions. A phosphate donor is a compound containing a phosphate moiety which the kinase is able to use to phosphorylate the protein substrate. ATP (adenosine-5'-triphosphate) is by far the most common phosphate donor. In certain instances, the sample will contain enough of a phosphate donor to make this step unnecessary. Then the fluorescent protein substrate is excited with light in its excitation spectrum. If the protein substrate has been phosphorylated, the substrate will exhibit different fluorescent properties, indicating that the sample contains protein kinase activity. For example, if the phosphorylated form of the protein substrate has higher fluorescence than the un-phosphorylated form, the amount of fluorescence in the sample will increase as a function of the amount of substrate that has been phosphorylated. If the fluorescent property is a change in the wavelength maximum of emission, the change will be detected as a decrease in fluorescence at the wavelength maximum of the un-phosphorylated substrate and an increase in fluorescence at the wavelength maximum of the phosphorylated substrate.

The amount of kinase activity in a sample can be determined by measuring the amount of a fluorescent property in the sample at a first time and a second time after contact between the sample, the fluorescent protein substrate and a phosphate donor, and determining the degree of change or the rate of change in a fluorescent property. For example, if phosphorylation results in an increase in fluorescence at the excitation wavelength maximum, the fluorescence of the substrate at this wavelength can be determined at two times. The amount of enzyme activity in the sample can be calculated as a function of the difference in the determined amount of the property at the two times. For example, the absolute amount of activity can be calibrated using standards of enzyme activity determined for certain amounts of enzyme after certain amounts of time. The faster or larger the difference in the amount, the more enzyme activity must have been present in the sample. The amount of a fluorescent property can be determined from any spectral or fluorescence lifetime characteristic of the excited substrate, for example, by determining the intensity of the fluorescent signal from the protein substrate or the excited state lifetime of the protein substrate, the ratio of the fluorescences at two different excitation wavelengths, the ratio of the intensities at two different emission wavelengths, or the excited lifetime of the protein substrate.

Fluorescence in a sample is measured using a fluorimeter. In general, excitation radiation from an excitation source having a first wavelength, passes through excitation optics. The excitation optics cause the excitation radiation to excite the sample. In response, fluorescent proteins in the sample emit radiation which has a wavelength that is different from the excitation wavelength. Collection optics then collect the emission from the sample. The device can include a temperature controller to maintain the sample at a specific temperature while it is being scanned. According to one embodiment, a multi-axis translation stage moves a microtiter plate holding a plurality of samples in order to position different wells to be exposed. The multi-axis translation stage, temperature controller, auto-focusing feature, and electronics associated with imaging and data collection can be managed by an appropriately programmed digital computer. The computer also can transform the data collected during the assay into another format for presentation. This process can be miniaturized and automated to enable screening many thousands of compounds.

Methods of performing assays on fluorescent materials are well known in the art and are described in, e.g., Lakowicz, J.R., Principles of Fluorescence Spectroscopy, New York:Plenum Press (1983); Herman, B., Resonance energy transfer microscopy, in: Fluorescence Microscopy of Living Cells in Culture, Part B, Methods in Cell Biology, vol. 30, ed. Taylor, D.L. & Wang, Y.-L., San Diego: Academic Press (1989), pp. 219-243; Turro, N.J., Modern Molecular Photochemistry, Menlo Park: Benjamin/Cummings Publishing Col, Inc. (1978), pp. 296-361.

Enzymatic assays also can be performed on isolated living cells *in vivo,* or from samples derived from organisms transfected to express the substrate. Because fluorescent protein substrates can be expressed recombinantly inside a cell, the amount of enzyme activity in the cell or organism of which it is a part can be determined by determining a fluorescent property or changes in a fluorescent property of cells or samples from the organism.

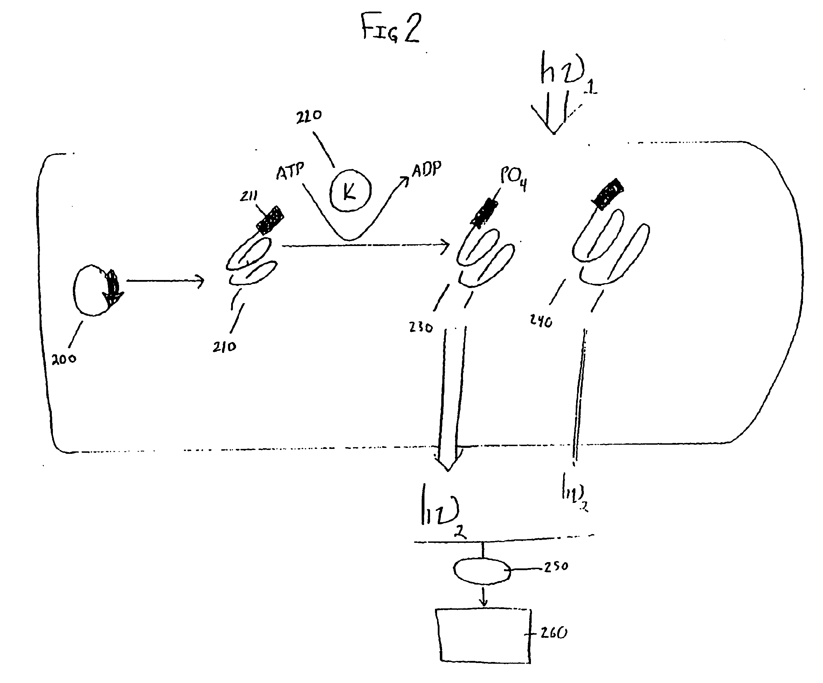

In one embodiment, shown in Fig 2, a cell is transiently or stably transfected with an expression vector 200 encoding a fluorescent protein substrate containing a phosphorylation site for the enzyme to be assayed. This expression vector optionally includes controlling nucleotide sequences such as promotor or enhancing elements. The expression vector expresses the fluorescent protein substrate 210 that contains the phosphorylation site 211 for the kinase to be detected. The enzyme to be assayed may either be intrinsic to the cell or may be introduced by stable transfection or transient co-transfection with another expression vector encoding the enzyme and optionally including controlling nucleotide sequences such as promoter or enhancer elements. The fluorescent protein substrate and the enzyme preferably are located in the same cellular compartment so that they have more opportunity to come into contact.

If the cell possesses a high degree of enzyme activity (K = "kinase" 220), the fluorescent protein substrate will be phosphorylated 230 (PO₄), usually through the hydrolysis of ATP. If the cell does not possess kinase activity, or possesses very little, the cell contains substantial amounts of un-phosphorylated substrate 240. Upon excitation with light of the appropriate wavelength (hv₁) the phosphorylated substrate will fluoresce light (hv₂). Un-phosphorylated substrate exhibits different fluorescent characteristics upon excitation at the same wavelength, and may, for example, not fluoresce at all, or fluoresce weakly. The amount of the fluorescent property is measured generally using the optics 250 and detector 260 of a fluorimeter.

If the cell contains phosphatases that compete with the protein kinases, removing the phosphate from the protein substrate, the level of enzyme activity in the cell can reach an equilibrium between phosphorylated and un-phosphorylated states of the protein substrate, and the fluorescence characteristics will reflect this equilibrium level. In one aspect, this method can be used to compare mutant cells to identify which ones possess greater or lesser ratio of kinase to phosphatase activity. Such cells can be sorted by a fluorescent cell sorter based on fluorescence.

A contemplated variation of the above assay is to use the controlling nucleotide sequences to produce a sudden increase in the expression of either the fluorescent protein substrate or the enzyme being assayed, e.g., by inducing expression of the construct. A fluorescent property is monitored at one or more time intervals after the onset of increased expression. A high amount of the property associated with phosphorylated state reflects a large amount or high efficiency of the kinase. This kinetic determination has the advantage of minimizing any dependency of the assay on the rates of degradation or loss of the fluorescent protein moieties.

In another embodiment, the vector may be incorporated into an entire organism by standard transgenic or gene replacement techniques. An expression vector capable of expressing the enzyme optionally may be incorporated into the entire organism by standard transgenic or gene replacement techniques. Then, a sample from the organism containing the protein substrate is tested. For example, cell or tissue homogenates, individual cells, or samples of body fluids, such as blood, can be tested.

### II. SCREENING ASSAYS

The enzymatic assays of the invention can be used in drug screening assays to determine whether a compound alters the activity of a protein kinase. In one embodiment, the assay is performed on a sample *in vitro* containing the enzyme. A sample containing a known amount of enzyme activity is mixed with a substrate of the invention and with a test compound. The amount of the enzyme activity in the sample is then determined as above, e.g., by measuring the amount of a fluorescent property at a first and second time after contact between the sample, the protein substrate, a phosphate substrate, and the compound. Then the amount of activity per mole of enzyme in the presence of the test compound is compared with the activity per mole of enzyme in the absence of the test compound. A difference indicates that the test compound alters the activity of the enzyme.

In another embodiment, the ability of a compound to alter kinase activity *in vivo* is determined. In an *in vivo* assay, cells transfected with a expression vector encoding a substrate of the invention are exposed to different amounts of the test compound, and the effect on fluorescence in each cell can be determined. Typically, the difference is calibrated against standard measurements to yield an absolute amount of kinase activity. A test compound that inhibits or blocks the activity or expression of the kinase can be detected by a relative increase in the property associated with the unphosphorylated state. The cell can also be transfected with an expression vector to co-express the kinase or an upstream signaling component such as a receptor, and fluorescent substrate. This method is useful for detecting signaling to a protein kinase of interest from an upstream component of the signaling pathway. If a signal from an upstream molecule, e.g., a receptor, is inhibited by a drug activity, then the kinase activity will not be altered from basal. This provides a method for screening for compounds which affect cellular events (including receptor-ligand binding, protein-protein interactions or kinase activation) which signal to the target kinase:

This invention also provides kits containing the fluorescent protein substrate and a phosphate substrate for the protein kinase. In one embodiment, the kit has a container holding the fluorescent protein substrate and another container holding the phosphate substrate. Protein kinases of known activity could be included for use as positive controls and standards.

### III. FLUORESCENT PROTEIN SUBSTRATES FOR PROTEIN KINASES

As used herein, the term "fluorescent property" refers to the molar extinction coefficient at an appropriate excitation wavelength, the fluorescence quantum efficiency, the shape of the excitation spectrum or emission spectrum, the excitation wavelength maximum and emission wavelength maximum, the ratio of excitation amplitudes at two different wavelengths, the ratio of emission amplitudes at two different wavelengths, the excited state lifetime, or the fluorescence anisotropy. A measurable difference in any one of these properties between the phosphorylated and unphosphorylated states suffices for the utility of the fluorescent protein substrates of the invention in assays for kinase activity. A measurable difference can be determined by determining the amount of any quantitative fluorescent property, e.g., the amount of fluorescence at a particular wavelength, or the integral of fluorescence over the emission spectrum. Optimally, the protein substrates are selected to have fluorescent properties that are easily distinguishable in the un-phosphorylated and phosphorylated states. Determining ratios of excitation amplitude or emission amplitude at two different wavelengths ("excitation amplitude ratioing" and "emission amplitude ratioing", respectively) are particularly advantageous because the ratioing process provides an internal reference and cancels out variations in the absolute brightness of the excitation source, the sensitivity of the detector, and light scattering or quenching by the sample. Furthermore, if phosphorylation of the protein substrate changes its ratio of excitation or emission amplitudes at two different wavelengths, then such ratios measure the extent of phosphorylation independent of the absolute quantity of the protein substrate. Some of the fluorescent protein substrates described herein do exhibit a phosphorylation-induced change in the ratio of excitation amplitudes at two different wavelengths. Even if a fluorescent protein substrate does not exhibit a phosphorylation-induced change in excitation or emission amplitudes at two wavelengths, cells can be provided that co-express another fluorescent protein that is not sensitive to phosphorylation and whose excitation or emission spectrum is peaked at wavelengths distinct from those of the phosphorylation substrate. Provided that the expression of the two proteins are both controlled by the same nucleotide control sequences, their expression levels should be closely linked. Therefore ratioing the excitation or emission amplitude of the phosphorylation substrate at its preferred wavelength to the corresponding excitation or emission amplitude of the phosphorylation-insensitive reference protein at its separate preferred wavelength is an alternative method for canceling out variations in the absolute quantity of cells or overall level of protein expression.

### A. Fluorescent Proteins

As used herein, the term "fluorescent protein" refers to any protein capable of fluorescence when excited with appropriate electromagnetic radiation. This includes fluorescent proteins whose amino acid sequences are either naturally occurring or engineered (i.e., analogs). Many cnidarians use green fluorescent proteins ("GFPs") as energy-transfer acceptors in bioluminescence. A "green fluorescent protein, as used herein, is a protein that fluoresces green light. Similarly, "blue fluorescent proteins" fluoresce blue light and "red fluorescent proteins" fluoresce red light. GFPs have been isolated from the Pacific Northwest jellyfish, *Aequorea victoria,* the sea pansy, *Renilla reniformis,* and *Phialidium gregarium.* W.W. Ward et al., Photochem. Photobiol., 35:803-808 (1982); L.D. Levine et al. , Comp. Biochem. Physiol., 72B:77-85 (1982).

A variety of *Aequorea-related* fluorescent proteins having useful excitation and emission spectra have been engineered by modifying the amino acid sequence of a naturally occurring GFP from *Aequorea victoria.* (D.C. Prasher et al., Gene, 111:229-233 (1992); R. Heim et al., Proc. Natl. Acad. Sci., USA, 91:12501-04 (1994); U.S. patent application 08/337,915, filed November 10, 1994; International application PCT/US95/14692, filed 11/10/95.)

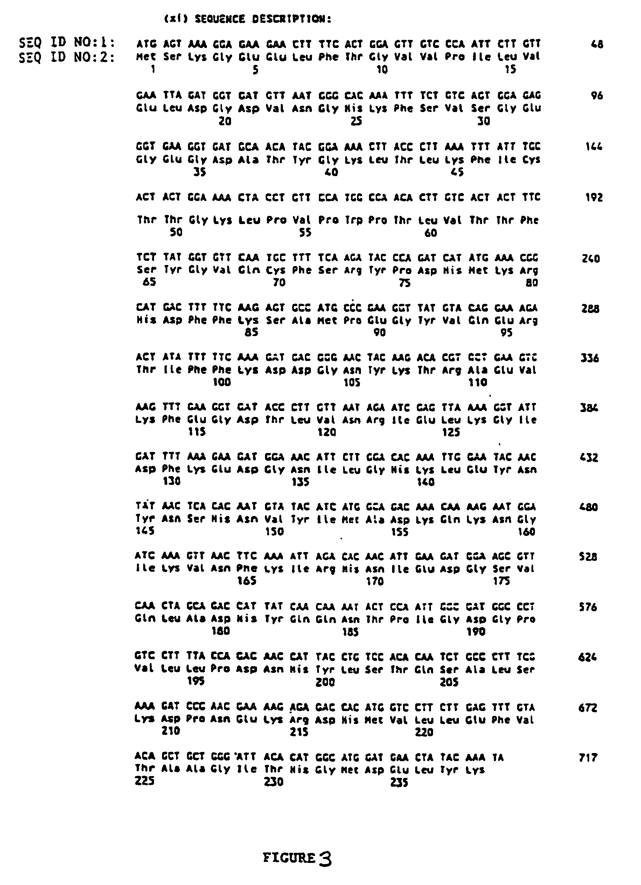

As used herein, a fluorescent protein is an "Aequorea-related fluorescent protein" if any contiguous sequence of 150 amino acids of the fluorescent protein has at least 85% sequence identity with an amino acid sequence, either contiguous or non-contiguous, from the 238 amino-acid wild-type *Aequorea* green fluorescent protein of Fig. 3 (SEQ ID NO:2). More preferably, a fluorescent protein is an *Aequorea*-related fluorescent protein if any contiguous sequence of 200 amino acids of the fluorescent protein has at least 95% sequence identity with an amino acid sequence, either contiguous or non-contiguous, from the wild type *Aequorea* green fluorescent protein of Fig. 3 (SEQ ID NO:2). Similarly, the fluorescent protein may be related to *Renilla* or *Phialidium* wild-type fluorescent proteins using the same standards.

Optimal alignment of sequences for aligning a comparison window may be conducted by the local homology algorithm of Smith and Waterman (1981) Adv. Appl. Math., 2:482, by the homology alignment algorithm of Needleman and Wunsch (1970) J. Mol. Biol., 48:443, by the search for similarity method of Pearson and Lipman (1988) Proc. Natl. Acad. Sci., U.S.A., 85:2444, by computerized implementations of these algorithms (GAP, BESTFIT, FASTA, and TFASTA in the Wisconsin Genetics Software Package Release 7.0, Genetics Computer Group, 575 Science Dr., Madison, WI), or by inspection. The best alignment (i.e., resulting in the highest percentage of homology over the comparison window, i.e., 150 or 200 amino acids) generated by the various methods is selected.

The percentage of sequence identity is calculated by comparing two optimally aligned sequences over the window of comparison, determining the number of positions at which the identical amino acid occurs in both sequences to yield the number of matched positions, dividing the number of matched positions by the total number of positions in the window of comparison (i.e., the window size), and multiplying the result by 100 to yield the percentage of sequence identity.

*Aequorea*-related fluorescent proteins include, for example and without limitation, wild-type (native) *Aequorea victoria* GFP (D.C. Prasher et al., "Primary structure of the Aequorea victoria green fluorescent protein," Gene, (1992) 111:229-33), whose nucleotide sequence (SEQ ID NO:1) and deduced amino acid sequence (SEQ ID NO:2) are presented in Fig. 3, allelic variants of this sequence, e.g., Q80R, which has the glutamine residue at position 80 substituted with arginine (M. Chalfie et al., Science, (1994) 263:802-805), those *Aequorea*-related engineered versions described in Table I, variants that include one or more folding mutations and fragments of these proteins that are fluorescent, such as *Aequorea* green fluorescent protein from which the two amino-terminal amino acids have been removed. Several of these contain different aromatic amino acids within the central chromophore and fluoresce at a distinctly shorter wavelength than wild type species. For example, mutants P4 and P4-3 contain (in addition to other mutations) the substitution Y66H, whereas W2 and W7 contain (in addition to other mutations) Y66W. Other mutations both close to the chromophore region of the protein and remote from it in primary sequence may affect the spectral properties of GFP and are listed in the first part of the table below.

**TABLE I**

| Clone | Mutation(s) | Excitation max (nm) | Emission max (nm) | Extinct. Coeff. (M⁻¹cm⁻¹) | Quantum yield |
|---|---|---|---|---|---|
| Wild type | none | 395 (475) | 508 | 21,000 (7,150) | 0.77 |
| P4 | Y66H | 383 | 447 | 13,500 | 0.21 |
| | | | | | |
| P4-3 | Y66H | 381 | 445 | 14,000 | 0.38 |
| | Y145F | | | | |
| | | | | | |
| W7 | Y66W | 433 (453) | 475 (501) | 18,000 | 0.67 |
| | N146I | | | (17,100) | |
| | M153T | | | | |
| | V163A | | | | |
| | N212K | | | | |
| | | | | | |
| W2 | Y66W | 432 (453) | 480 | 10,000 (9,600) | 0.72 |
| | I123V | | | | |
| | Y145H | | | | |
| | H148R | | | | |
| | M153T | | | | |
| | V163A | | | | |
| | N212K | | | | |
| | | | | | |
| S65T | S65T | 489 | 511 | 39,200 | 0.68 |
| | | | | | |
| P4-1 | S65T | 504 (396) | 514 | 14,500 (8,600) | 0.53 |
| | M153A | | | | |
| | K238E | | | | |
| | | | | | |
| S65A | S65A | 471 | 504 | | |
| | | | | | |
| S65C | S65C | 479 | 507 | | |
| | | | | | |
| S65L | S65L | 484 | 510 | | |
| | | | | | |
| Y66F | Y66F | 360 | 442 | | |
| | | | | | |
| Y66W | Y66W | 458 | 480 | | |

Additional mutations in *Aequorea-related* fluorescent proteins, referred to as "folding mutations," improve the ability of GFP to fold at higher temperatures, and to be more fluorescent when expressed in mammalian cells, but have little or no effect on the peak wavelengths of excitation and emission. It should be noted that these may be combined with mutations that influence the spectral properties of GFP to produce proteins with altered spectral and folding properties. Folding mutations include: T44A, F64L, V68L, S72A, F99S, Y145F, N146I, M153T or A, V163A, I167T, S175G, S205T and N212K.

This invention contemplates the use of other fluorescent proteins in fluorescent protein substrates for protein kinases. The cloning and expression of yellow fluorescent protein from *Vibrio fischeri* strain Y-1 has been described by T.O. Baldwin et al., Biochemistry (1990) 29:5509-15. This protein requires flavins as fluorescent co-factors. The cloning of Peridinin-chlorophyll a binding protein from the dinoflagellate *Symbiodinium* sp. was described by B. J. Morris et al., Plant Molecular Biology, (1994) 24:673:77. One useful aspect of this protein is that it fluoresces red. The cloning of phycobiliproteins from marine cyanobacteria such as *Synechococcus,* e.g., phycoerythrin and phycocyanin, is described in S.M. Wilbanks et al., J. Biol. Chem. (1993) 268: 1226-35. These proteins require phycobilins as fluorescent co-factors, whose insertion into the proteins involves auxiliary enzymes. The proteins fluoresce at yellow to red wavelengths.

As used herein, the "fluorescent protein moiety" of a fluorescent protein substrate is that portion of the amino acid sequence of a fluorescent protein substrate which, when the amino acid sequence of the fluorescent protein substrate is optimally aligned with the amino acid sequence of a naturally occurring fluorescent protein, lies between the amino terminal and carboxy terminal amino acids, inclusive, of the amino acid sequence of the naturally occurring fluorescent protein.

It has been found that fluorescent proteins can be genetically fused to other target proteins and used as markers to identify the location and amount of the target protein produced. Accordingly, this invention provides fusion proteins comprising a fluorescent protein moiety and additional amino acid sequences. Such sequences can be, for example, up to about 15, up to about 50, up to about 150 or up to about 1000 amino acids long. The fusion proteins possess the ability to fluoresce when excited by electromagnetic radiation. In one embodiment, the fusion protein comprises a polyhistidine tag to aid in purification of the protein.

### B. Phosphorylation Sites For Protein Kinases

Fluorescent protein substrates for a protein kinase are the subset of fluorescent proteins as defined above whose amino acid sequence includes a phosphorylation site. Fluorescent protein substrates can be made by modifying the amino acid sequence of an existing fluorescent protein to include a phosphorylation site for a protein kinase. Fluorescent protein substrates for protein kinases are not meant to include isolated fluorescent proteins that have a naturally occurring phosphorylation site, naturally occurring fluorescent proteins or currently known mutant fluorescent proteins. Such previously known fluorescent proteins or mutants may be substrates for protein kinases, but do not exhibit any detectable change in fluorescent properties upon phosphorylation.

As used herein, the term "phosphorylation site for a protein kinase" refers to an amino acid sequence which, as part of a polypeptide, is recognized by a protein kinase for the attachment of a phosphate moiety. The phosphorylation site can be a site recognized by, for example, protein kinase A, a cGMP-dependent protein kinase, protein kinase C, Ca²⁺/calmodulin-dependent protein kinase I, Ca²⁺/ calmodulin-dependent protein kinase II or MAP kinase activated protein kinase type 1.

The preferred consensus sequence for protein kinase A is RRXSZ (SEQ ID NO:3) or RRXTZ (SEQ ID NO:4), wherein X is any amino acid and Z is a hydrophobic amino acid, preferably valine, leucine or isoleucine. Many variations in the above sequence are allowed, but generally exhibit poorer kinetics. For example, lysine (K) can be substituted for the second arginine. Many consensus sequences for other protein kinases have been tabulated, e.g. by Kemp, B.E. and Pearson, R.B. (1990) Trends Biochem. Sci. 15: 342-346; Songyang, Z. et al. (1994) Current Biology 4: 973-982.

For example, a fluorescent protein substrate selective for phosphorylation by cGMP-dependent protein kinase can include the following consensus sequence: BKISASEFDR PLR (SEQ ID NO:5), where B represents either lysine (K) or arginine (R), and the first S is the site of phosphorylation (Colbran et al. (1992) J. Biol. Chem. 267: 9589-9594). The residues DRPLR (SEQ ID NO:6) are less critical than the phenylalanine (F) just preceding them for specific recognition by cGMP-dependent protein kinase in preference to cAMP-dependent protein kinase.

Either synthetic or naturally occurring motifs can be used to create a protein kinase phosphorylation site. For example, peptides including the motif XRXXSXRX (SEQ ID NO:7), wherein X is any amino acid, are among the best synthetic substrates (Kemp and Pearson, *supra*) for protein kinase C. Alternatively, the Myristoylated Alanine-Rich Kinase C substrate ("MARCKS") is one of the best substrates for PKC and is a real target for the kinase *in vivo.* The sequence around the phosphorylation site of MARCKS is KKKKRFSFK (SEQ ID NO:8) (Graff et al. (1991) J. Biol. Chem. 266:14390-14398). Either of these two sequences can be incorporated into a fluorescent protein to make it a substrate for protein kinase C.

A protein substrate for Ca²⁺/calmodulin-dependent protein kinase I is derived from the sequence of synapsin I, a known optimal substrate for this kinase. The recognition sequence around the phosphorylation site is LRRLSDSNF (SEQ ID NO:9) (Lee et al. (1994) Proc. Natl. Acad. Sci. USA 91:6413-6417).

A protein substrate selective for Ca²⁺/calmodulin-dependent protein kinase II is derived from the sequence of glycogen synthase, a known optimal substrate for this kinase. The recognition sequence around the phosphorylation site is KKLNRTLTVA (SEQ ID NO:10) (Stokoe et al. (1993) Biochem. J. 296:843-849). A small change in this sequence to KKANRTLSVA (SEQ ID NO:11) makes the latter specific for MAP kinase activated protein kinase type 1.

In one embodiment, the fluorescent protein substrate contains a phosphorylation site around one of the termini, in particular, the amino-terminus, of the fluorescent protein moiety. The site preferably is located in a position within five, ten, fifteen, or twenty amino acids of a position corresponding to the wild type amino-terminal amino acid of the fluorescent protein moiety ("within twenty amino acids of the amino-terminus"). This includes sites engineered into the existing amino acid sequence of the fluorescent protein moiety and sites produces by extending the amino terminus of the fluorescent protein moiety.

One may, for example, modify the existing sequence of wild type *Aequorea* GFP or a variant or it as listed above to include a phosphorylation site within the first ten or twenty amino acids. In one embodiment, the naturally occurring sequence is modified as follows:

| | | |
|---|---|---|
| wild type: | MSKGEELFTG | (1-10 of SEQ ID NO:2) |
| substrate: | MRRRRSIITG | (SEQ ID NO:12). |

One may include modifying the naturally occurring sequence of *Aequorea* GFP by introducing a phosphorylation site into an extended amino acid sequence of such a protein created by adding flanking sequences to the amino terminus, for example:

| | | |
|---|---|---|
| wild type: | MSKGEELFTG | (1-10 of SEQ ID NO:2) |
| substrate: | MRRRRSIIIIFTG | (SEQ ID NO: 13). |

Fluorescent protein substrates having a phosphorylation site around a terminus of the fluorescent protein moiety offer the following advantages. First, it is often desirable to append additional amino acid residues onto the fluorescent protein moiety in order to create a specific phosphorylation consensus sequence. Such a sequence is much less likely to disrupt the folding pattern of the fluorescent protein when appended onto the terminus than when inserted into the interior of the protein sequence. Second, different phosphorylation motifs can be interchanged without significant disruption of GFP therefore providing a general method of measuring different kinases. Third, the phosphorylation site is exposed to the surface of the protein and, therefore, more accessible to protein kinases. Fourth, we have discovered that phosphorylation at sites close to the N-terminus of GFP can provide large changes in fluorescent properties if the site of phosphorylation is chosen such that the Ser or Thr residue which is phosphorylated occupies a position which in the wild-type protein was originally negatively or positively charged. Specifically, replacement of Glu 5 or Glu 6 by a non-charged Ser or Thr residue can significantly disrupt fluorescence of GFP when made within the right context of surrounding amino acids. Phosphorylation of the serine or threonine will restore negative charge to this position and thereby increases fluorescence.

In another embodiment, the fluorescent protein substrate includes a phosphorylation site remote from the terminus, e.g., that is separated by more than about twenty amino acids from the terminus of the fluorescent protein moiety and within the fluorescent protein moiety. One embodiment of this form includes the *Aequorea-*related fluorescent protein substrate comprising the substitution H217S, creating a consensus protein kinase A phosphorylation site. Additionally, phosphorylation sites comprising the following alterations based on the sequence of wild type *Aequorea* GFP exhibit fluorescent changes upon phosphorylation: 69RRFSA (SEQ ID NO:14) and 214KRDSM (SEQ ID NO:15).

The practitioner should consider the following in selecting amino acids for substitution within the fluorescent protein moiety remote in primary amino acid sequence from the terminus. First, it is preferable to select amino acid sequences within the fluorescent protein moiety that resemble the sequence of the phosphorylation site. In this way, fewer amino acid substitutions in the native protein are needed to introduce the phosphorylation site into the fluorescent protein. For example, protein kinase A recognizes the sequence RRXSZ (SEQ ID NO:3) or RRXTZ (SEQ ID NO:4), wherein X is any amino acid and Z is a hydrophobic amino acid. Serine or threonine is the site of phosphorylation. It is preferable to introduce this sequence into the fluorescent protein moiety at sequences already containing Ser or Thr, so that Ser or Thr are not substituted in the protein. More preferably the phosphorylation site is created at locations having some existing homology to the sequence recognized by protein kinase A, e.g., having a proximal Arg or hydrophobic residues with the same spatial relationship as in the phosphorylation site.

Second, locations on the surface of the fluorescent protein are preferred for phosphorylation sites. This is because surface locations are more likely to be accessible to protein kinases than interior locations. Surface locations can be identified by computer modeling of the fluorescent protein structure or by reference to the crystal structure of *Aequorea* GFP. Also, charged amino acids in the fluorescent protein are more likely to lie on the surface than inside the fluorescent protein, because such amino acids are more likely to be exposed to water in the environment.

In cases where the phosphorylation site is either at the N-terminus or remote from it, the amino acid context around the phosphorylation site needs to be optimized in order to maximize the change in fluorescence. Amino acid substitutions that change large bulky and or hydrophobic amino acids to smaller and less hydrophobic replacements are generally helpful. Similarly large charged amino acids can be replaced by smaller, less charged amino acids. For example:
a/Hydrophobic to less hydrophobic
   Phe to Leu
   Leu to Ala
b/Charged to charged but smaller
   Glu to Asp
   Arg to Lys
c/Charged to less charged
   Glu to Gln
   Asp to Asn
d/Charged to polar
   Glu to Thr
   Asp to Ser
e/Charged to non-polar
   Glu to Leu
   Asp to Ala

These changes can be accomplished by directed means or using random iterative approaches where changes are made randomly and the best ones selected based upon their change in fluorescent properties after phosphorylation by an appropriate kinase.

Third, amino acids at distant locations from the actual site of phosphorylation can be varied to enhance fluorescence changes upon phosphorylation. These mutations can be created through site directed mutagenesis, or through random mutagenesis, for example by error-prone PCR, to identify mutations that enhance either absolute fluorescence or the change in fluorescence upon phosphorylation. The identification of mutants remote in primary sequence from the N-terminus identifies potentially interacting sequences which may provide additional areas in which further mutagenesis could be used to refine the change in fluorescence upon phosphorylation. For example, it has been determined that mutations around the amino terminus phosphorylation site interact (either transiently during folding, or in a stable fashion) with amino acids at positions 171 and 172, and that point mutations that significantly disrupt fluorescence of GFP by changing negative to positive charges near the amino terminus can be rescued by changing a positive to a negative charge at position 171.

In the phosphorylation mutant 50 the sequence is a/ and for reference the wild type sequence b/ is listed below.
a/ MSKRRDSLT (SEQ ID NO:16)
b/ MSKGEELFT (1-9 of SEQ ID NO:2)

The phosphorylation mutant has only 7% of the fluorescence of wild type protein. However, its fluorescence can be restored to 80% of wild type by 2 amino acid changes, E171K and I172V, positions which are quite remote in linear sequence from the amino terminus.

Thus, changes in charge at E171K (negative to positive) can almost completely restore the fluorescence of the phosphorylation mutant, strongly suggesting that the original loss of fluorescence arose primarily through changes in charge caused by the point mutations. It is clear that the addition and loss of charge at positions around, and at the phosphorylation site, have a significant impact on fluorescence formation. The fact that charge alone can significantly affect the fluorescence properties of GFP is highly significant within the scope of the present application since phosphorylation involves the addition of 2 negative charges associated with the phosphate group (OPO₃⁻²) on the serine residue.

In the above case the mutations restore fluorescence of the phosphorylation mutant, without significantly increasing the magnitude of the change in fluorescence upon phosphorylation. Nevertheless the identification of these positions in GFP provides a valuable tool to further optimize changes in fluorescence upon phosphorylation by creating random mutations at codons around positions 171, 172 and 173 to identify mutations that enhance changes in fluorescence upon phosphorylation.

This can be achieved by co-expressing the kinase of interest with the fluorescent substrate of the invention containing random mutations which may enhance the fluorescence changes upon phosphorylation in bacteria (in the example above these would be NNK mutations at codons 171, 172 and 173, where N represents a random choice of any of the four bases and K represents a random choice of guanine or thymine). The expression vector containing the mutated fluorescent substrates and the kinase are transformed into host bacteria and the individual bacterial colonies grown up. Each colony is derived from a single cell, and hence contains a single unique mutant fluorescent substrate grown up.

The individual colonies may then be grown up and screened for fluorescence either by fluorescence activated cell sorting (FACS), or by observation under a microscope. Those that exhibit the greatest fluorescence can then be rescreened under conditions in which the kinase gene is inactivated. This can be achieved by appropriate digests of the kinase gene by restriction enzymes that specifically cut within the kinase but not GFP. Comparison of the brightness of the mutant first in the presence of kinase then in its absence indicates the relative effect of phosphorylation on the mutant GFP.

### C. Production Of Fluorescent Protein Substrates For Protein Kinases

While certain fluorescent protein substrates for protein kinases can be prepared chemically, for example, by coupling a peptide moiety to the amino terminus of a fluorescent protein, it is preferable produce fluorescent protein substrates recombinantly.

Recombinant production of a fluorescent protein substrate involves expressing a nucleic acid molecule having sequences that encode the protein. As used herein, the term "nucleic acid molecule" includes both DNA and RNA molecules. It will be understood that when a nucleic acid molecule is said to have a DNA sequence, this also includes RNA molecules having the corresponding RNA sequence in which "U" replaces "T." The term "recombinant nucleic acid molecule" refers to a nucleic acid molecule which is not naturally occurring, and which comprises two nucleotide sequences which are not naturally joined together. Recombinant nucleic acid molecules are produced by artificial combination, e.g., genetic engineering techniques or chemical synthesis.

In one embodiment, the nucleic acid encodes a fusion protein in which a single polypeptide includes the fluorescent protein moiety within a longer polypeptide. In another embodiment the nucleic acid encodes the amino acid sequence of consisting essentially of a fluorescent protein modified to include a phosphorylation site. In either case, nucleic acids that encode fluorescent proteins are useful as starting materials.

Nucleic acids encoding fluorescent proteins can be obtained by methods known in the art. For example, a nucleic acid encoding a green fluorescent protein can be isolated by polymerase chain reaction of cDNA from *A. victoria* using primers based on the DNA sequence of *A. victoria* green fluorescent protein, as presented in Fig. 3. PCR methods are described in, for example, U.S. Pat. No. 4,683,195; Mullis et al. (1987) Cold Spring Harbor Symp. Quant. Biol. 51:263; and Erlich, ed., PCR Technology, (Stockton Press, NY, 1989).

Mutant versions of fluorescent proteins can be made by site-specific mutagenesis of other nucleic acids encoding fluorescent proteins, or by random mutagenesis caused by increasing the error rate of PCR of the original polynucleotide with 0.1 mM MnCl₂ and unbalanced nucleotide concentrations. See, e.g., U.S. patent application 08/337,915, filed November 10, 1994 or International application PCT/US95/14692, filed 11/10/95.

Nucleic acids encoding fluorescent protein substrates which are fusions between a polypeptide including a phosphorylation site and a fluorescent protein and can be made by ligating nucleic acids that encode each of these. Nucleic acids encoding fluorescent protein substrates which include the amino acid sequence of a fluorescent protein in which one or more amino acids in the amino acid sequence of a fluorescent protein are substituted to create a phosphorylation site can be created by, for example, site specific mutagenesis of a nucleic acid encoding a fluorescent protein.

The construction of expression vectors and the expression of genes in transfected cells involves the use of molecular cloning techniques also well known in the art. Sambrook et al., Molecular Cloning - A Laboratory Manual, Cold Spring Harbor Laboratory, Cold Spring Harbor, NY, (1989) and Current Protocols in Molecular Biology, F.M. Ausubel et al., eds., (Current Protocols, a joint venture between Greene Publishing Associates, Inc. and John Wiley & Sons, Inc.

Nucleic acids used to transfect cells with sequences coding for expression of the polypeptide of interest generally will be in the form of an expression vector including expression control sequences operatively linked to a nucleotide sequence coding for expression of the polypeptide. As used, the term nucleotide sequence "coding for expression of" a polypeptide refers to a sequence that, upon transcription and translation of mRNA, produces the polypeptide. As any person skilled in the art recognizes, this includes all degenerate nucleic acid sequences encoding the same amino acid sequence. This can include sequences containing, e.g., introns. As used herein, the term "expression control sequences" refers to nucleic acid sequences that regulate the expression of a nucleic acid sequence to which it is operatively linked. Expression control sequences are "operatively linked" to a nucleic acid sequence when the expression control sequences control and regulate the transcription and, as appropriate, translation of the nucleic acid sequence. Thus, expression control sequences can include appropriate promoters, enhancers, transcription terminators, a start codon (i.e., ATG) in front of a protein-encoding gene, splicing signals for introns, maintenance of the correct reading frame of that gene to permit proper translation of the mRNA, and stop codons.

The recombinant nucleic acid can be incorporated into an expression vector comprising expression control sequences operatively linked to the recombinant nucleic acid. The expression vector can be adapted for function in prokaryotes or eukaryotes by inclusion of appropriate promoters, replication sequences, markers, etc.

The expression vector can be transfected into a host cell for expression of the recombinant nucleic acid. Host cells can be selected for high levels of expression in order to purify the protein. *E. coli* is useful for this purpose. Alternatively, the host cell can be a prokaryotic or eukaryotic cell selected to study the activity of an enzyme produced by the cell. The cell can be, e.g., a cultured cell or a cell *in vivo.*

Recombinant fluorescent protein substrates can be produced by expression of nucleic acid encoding for the protein in *E. coli. Aequorea-*related fluorescent proteins are best expressed by cells cultured between about 15° C and 30° C but higher temperatures (e.g. 37° C) are possible. After synthesis, these enzymes are stable at higher temperatures (e.g., 37° C) and can be used in assays at those temperatures.

The construct can also contain a tag to simplify isolation of the substrate. For example, a polyhistidine tag of, e.g., six histidine residues, can be incorporated at the amino or carboxyl terminal of the fluorescent protein substrate. The polyhistidine tag allows convenient isolation of the protein in a single step by nickel-chelate chromatography.

Alternatively, the substrates need not be isolated from the host cells. This method is particularly advantageous for the assaying for the presence of protein kinase activity *in situ.*

### IV. LIBRARIES OF CANDIDATE SUBSTRATES

The inclusion of a phosphorylation site around the amino terminus of a fluorescent protein moiety can provide a fluorescent protein that, when phosphorylated, can alter a fluorescent property of the protein. Accordingly, this invention provides libraries of fluorescent protein candidate substrates useful for screening in the identification and characterization of sequences that can be recognized and efficiently phosphorylated by a kinase. Libraries of these proteins can be screened to identify sequences that can be phosphorylated by kinases of unknown substrate specificity, or to characterize differences in kinase activity in, or from, diseased and normal cells or tissues.

As used herein, a "library" refers to a collection containing at least 10 different members. Each member of a fluorescent protein candidate substrate library comprises a fluorescent protein moiety and a variable peptide moiety, which is preferably located near the amino-terminus of the fluorescent protein moiety and preferably has fewer than about 15 amino acids. The variety of amino acid sequences for the peptide moiety is at the discretion of the practitioner. For example, the library can contain a quite diverse collection of variable peptide moieties in which most or all of the amino acid positions are subjected to a non-zero but low probability of substitution. Also, the library can contain variable peptide moieties having an amino acid sequence in which only a few, e.g., one to ten, amino acid positions are varied, but the probability of substitution at each position is relatively high.

Preferably, libraries of fluorescent protein candidate substrates are created by expressing protein from libraries of recombinant nucleic acid molecules having expression control sequences operatively linked to nucleic acid sequences that code for the expression of different fluorescent protein candidate substrates. Methods of making nucleic acid molecules encoding a diverse collection of peptides are described in, for example, U.S. patent 5,432,018 (Dower et al.), U.S. patent 5,223,409 (Ladner et al.), U.S. Patent 5,264,563 (Huse), and International patent publication WO 92/06176 (Huse et al.). For expression of fluorescent protein candidate substrates, recombinant nucleic acid molecules are used to transfect cells, such that each cell contains a member of the library. This produces, in turn, a library of host cells capable of expressing the library of different fluorescent protein candidate substrates. The library of host cells is useful in the screening methods of this invention.

In one method of creating such a library, a diverse collection of oligonucleotides having preferably random codon sequences are combined to create polynucleotides encoding peptides having a desired number of amino acids. The oligonucleotides preferably are prepared by chemical synthesis. The polynucleotides encoding variable peptide moiety can then be coupled to the 5' end of a nucleic acid coding for the expression of a fluorescent protein moiety or a carboxy-terminal portion of it. That is, the fluorescent protein moiety can be cut back to eliminate up to 20 amino acids of the reference fluorescent protein. This creates a recombinant nucleic acid molecule coding for the expression of a fluorescent protein candidate substrate having a peptide moiety fused to the amino terminus of the fluorescent protein. This recombinant nucleic acid molecule is then inserted into an expression vector to create a recombinant nucleic acid molecule comprising expression control sequences operatively linked to the sequences encoding the candidate substrate.

To generate the collection of oligonucleotides which forms a series of codons encoding a random collection of amino acids and which is ultimately cloned into the vector, a codon motif is used, such as (NNK)ₓ, where N may be A, C, G, or T (nominally equimolar), K is G or T (nominally equimolar), and x is the desired number of amino acids in the peptide moiety, e.g., 15 to produce a library of 15-mer peptides. The third position may also be G or C, designated "S". Thus, NNK or NNS (i) code for all the amino acids, (ii) code for only one stop codon, and (iii) reduce the range of codon bias from 6:1 to 3:1. The expression of peptides from randomly generated mixtures of oligonucleotides in appropriate recombinant vectors is discussed in Oliphant et al., Gene 44:177-183 (1986).

An exemplified codon motif (NNK)₆ (SEQ ID NO:17) produces 32 codons, one for each of 12 amino acids, two for each of five amino acids, three for each of three amino acids and one (amber) stop codon. Although this motif produces a codon distribution as equitable as available with standard methods of oligonucleotide synthesis, it results in a bias against peptides containing one-codon residues.

An alternative approach to minimize the bias against one-codon residues involves the synthesis of 20 activated tri-nucleotides, each representing the codon for one of the 20 genetically encoded amino acids. These are synthesized by conventional means, removed from the support but maintaining the base and 5-HO-protecting groups, and activating by the addition of 3' O-phosphoramidite (and phosphate protection with beta-cyanoethyl groups) by the method used for the activation of mononucleosides, as generally described in McBride and Caruthers, Tetrahedron Letters 22:245 (1983). Degenerate "oligocodons" are prepared using these trimers as building blocks. The trimers are mixed at the desired molar ratios and installed in the synthesizer. The ratios will usually be approximately equimolar, but may be a controlled unequal ratio to obtain the over- to under-representation of certain amino acids coded for by the degenerate oligonucleotide collection. The condensation of the trimers to form the oligocodons is done essentially as described for conventional synthesis employing activated mononucleosides as building blocks. See generally, Atkinson and Smith, Oligonucleotide Synthesis, M.J. Gait, ed. p35-82 (1984). Thus, this procedure generates a population of oligonucleotides for cloning that is capable of encoding an equal distribution (or a controlled unequal distribution) of the possible peptide sequences.

Libraries of amino terminal phosphorylation sites may also be annealed to libraries of randomly mutated GFP sequences to enable the selection of optimally responding substrates.

### V. METHODS FOR SCREENING LIBRARIES OF CANDIDATE SUBSTRATES

Libraries of host cells expressing fluorescent protein candidate substrates are useful in identifying fluorescent proteins having peptide moieties that alter a fluorescent property of the fluorescent protein. Several methods of using the libraries are envisioned. In general, one begins with a library of recombinant host cells, each of which expresses a different fluorescent protein candidate substrate. Each cell is expanded into a clonal population that is genetically homogeneous.

In a first method, the desired fluorescent property is measured from each clonal population before and at least one specified time after a known change in intracellular protein kinase activity. This change in kinase activity could be produced by transfection with a gene encoding the kinase, by induction of kinase gene expression using expression control elements, or by any condition that post-translationally modulates activity of a kinase that has already been expressed. Examples of the latter include cell surface receptor mediated elevation of intracellular cAMP to activate cAMP-dependent surface receptor mediated increases of intracellular cGMP to activate cGMP-dependent protein kinase, cytosolic free calcium to activate Ca²⁺/calmodulin-dependent protein kinase types I, II, or IV, or the production of diacylglycerol to activate protein kinase C, etc. One then selects for the clone(s) that show the biggest or fastest change in the desired fluorescence property. This method detects fluorescent protein mutants whose folding and maturation was influenced by phosphorylation as well as those affected by phosphorylation after maturation.

One embodiment of this method exploits the fact that the catalytic subunit of cAMP-dependent protein kinase is constitutively active in the absence of the regulatory subunit and is growth-inhibitory in *E. coli* and most mammalian cells. Therefore, the cells tend to shed the kinase gene by recombination. The change in kinase activity is obtained by culturing the cells for a time sufficient to lose the kinase gene.

In a second method the host cells do not express the protein kinase of interest. Each clonal population is separately lysed. ATP is then added to the lysate. After an incubation period to allow phosphorylation by background kinases, the fluorescence property is measured. Then exogenous protein kinase is added to the lysate and the fluorescent property is re-measured at one or more specified time points. Again one selects for the clone(s) that show the biggest or fastest change(s) in the desired fluorescence property. Because little or no fresh protein synthesis is likely to occur in the lysate, this method would discriminate against mutants which are sensitive to phosphorylation only during their folding and maturation.

In one embodiment of this method, the lysate is split into two aliquots, one of which is mixed with kinase and ATP, the other of which receives only ATP. One selects for the clone(s) that show the biggest difference in fluorescence property between the two aliquots.

The nucleic acids from cells exhibiting the different properties can be isolated from the cells. Candidate substrates having different fluorescent properties can be tested further to identify the source of the difference.

The host cell also can be transfected with an expression vector capable of expressing an enzyme, such as a protein kinase, whose effect on the fluorescent property is to be tested.

### VI. EXAMPLES

### A. Phosphorylation sites located in the amino acid sequence of Aequorea GFP remote in the primary amino acid sequence from the N-terminus

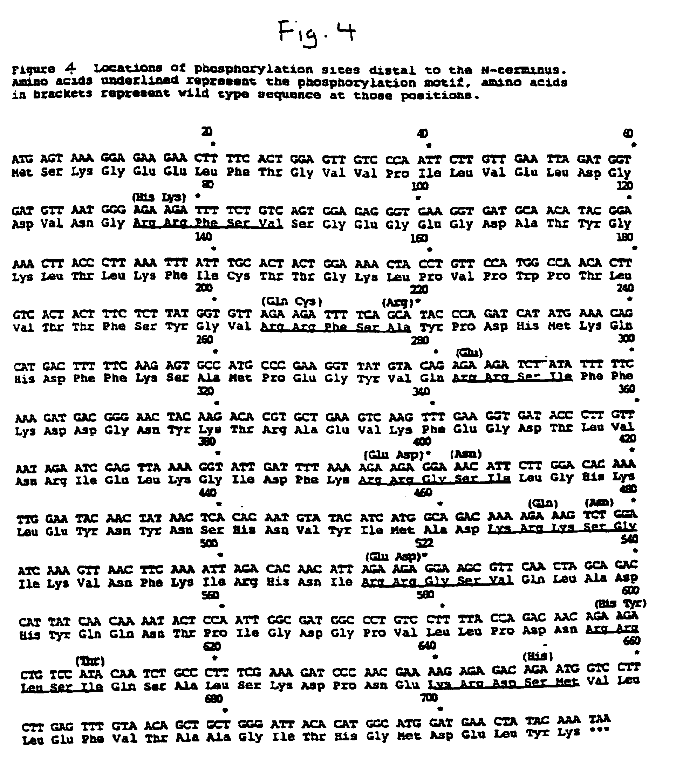

Potential sites for phosphorylation were chosen at or close to positions in GFP which had previously been identified to exert significant effects on fluorescence, or which had a higher probability of surface exposure based on computer algorithms (Fig. 4). For example, in a mutant called H9, Ser202 and Thr203 are mutated to F and I respectively, creating a large change in spectral properties (see also Ehrig *et al,* 1995). Therefore in one mutant, 199RRLSI (SEQ ID NO:18), a potential site of phosphorylation was created around Ser202, whose phosphorylation should significantly affect the fluorescent properties. Similarly the amino acids located at positions 72 and 175 have been implicated in increased folding efficiency of GFP at higher temperatures and were made into potential sites of phosphorylation in separate mutants.

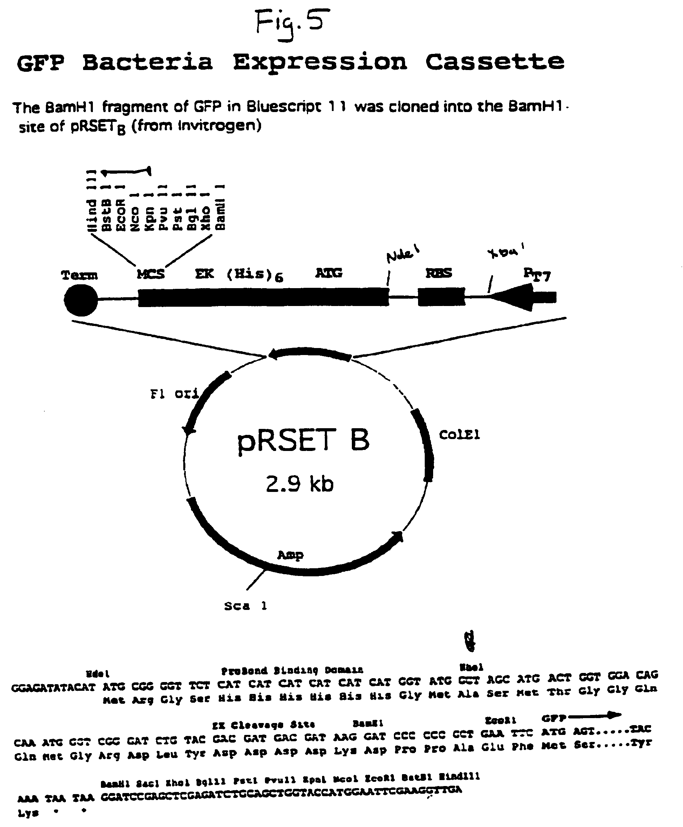

A complete list of the positions and amino acid changes made for each phosphorylation mutant in this series is outlined in Fig. 4. GFP was expressed in *E. coli* using the expression plasmid pRSET (Invitrogen), in which the region encoding GFP was fused in frame with nucleotides encoding an N-terminal polyhistidine tag (Fig. 5). The sequence changes were introduced by site-directed mutagenesis using the Bio-Rad mutagenesis kit (Kunkel, T.A. (1985) Proc. Natl. Acad. Sci. 82:488-492, Kunkel,T.A., Roberts, J.D., and Zakour, R.A. (1987) Meth Enzymol 154:367-382) and confirmed by sequencing. The recombinant proteins were induced with IPTG and expressed in bacteria and purified by nickel affinity chromatography. The sequence changes, relative fluorescence, relative rate of phosphorylation and % change in fluorescence upon phosphorylation are listed in Table II. In those cases where the protein exhibited no fluorescence after insertion of the phosphorylation site no determinations were made on the effect of phosphorylation on fluorescence.

**Table II: Relative fluorescence, rate of phosphorylation and change in fluorescence upon phosphorylation for mutants incorporating phosphorylation sites remote from the N-terminus**

| SEQ ID NO: | Sequence | Fluorescence before phosphorylation (% of wild type) | Relative rates of phosphorylation | %Change in fluorescence after incubation with kinase |
|---|---|---|---|---|
| 19 | 25RRFSV | 95 | 1.75 | -5 |
| 20 | 68RRFSR | 0 | n.d | n.d |
| 14 | 68RRFSA | 6 | 0.6 | +10 |
| 21 | 94RRSIF | 0 | n.d | n.d |
| 22 | 131RRGSIL | 0 | n.d | n.d |
| 23 | 155KRKSGI | 86 | 2.5 | 0 |
| 24 | 172RRGSV | 90 | 1.57 | 0 |
| 18 | 199RRLSI | 0 | n.d | n.d |
| 15 | 214KRDSM | 21 | 1.88 | +40 |

Bold letters indicate site of phosphorylation. Numbers prior to the sequence indicate amino acid position in wild type GFP (Fig. 3, SEQ ID NO:2) where phosphorylation site starts. The relative rates of phosphorylation compare the rate of phosphorylation of the given phosphorylation site with the endogenous protein kinase A phosphorylation site in *Aequorea* GFP (HKFSV SEQ ID NO:1) measured by incorporation of ³²P after incubation of the purified substrate and protein kinase A catalytic subunit in the presence of ³²P-labelled ATP using 3µg GFP, 5µg protein kinase A catalytic subunit for 10 minutes at 30°C in standard phosphorylation buffer (20 mM MOPS pH 6.5, 100mM KCl, 100µM ATP, 3mM MgCl₂ 1 mM DTT and 100uCi ³²P-labeled ATP. Reactions were terminated by blotting onto phosphocellulose paper and washing with 10% phosphoric acid. The % change in fluorescence represents the increase in fluorescence (475 nm excitation, 510 nm emission) observed in each purified protein resulting from incubation with excess protein kinase A catalytic subunit for 1 hour at 30° C using the same phosphorylation conditions as described above except that no ³²P-labeled ATP was present and that after the reaction time was complete samples were analyzed in the fluorimeter rather than blotted onto phosphocellulose paper.

The greatest changes in fluorescence occurred in mutant 214KRDSM (SEQ ID NO:15) which exhibited a 40 % change in fluorescence upon phosphorylation. However analysis of the kinetics of phosphorylation using γ-³²P-labeled ATP demonstrated that the site is poorly phosphorylated by protein kinase A. Wild type GFP contains a mediocre consensus phosphorylation site (25HKFSV, from SEQ ID NO:1) that can be phosphorylated by protein kinase A *in vitro* with relatively slow kinetics. While phosphorylation at this position has no detectable effect on the fluorescence of GFP, the rate of phosphorylation at this position is used as an internal control between experiments to determine the relative rates of phosphorylation at sites engineered into the protein by site directed mutagenesis.

### B. Phosphorylation sites around the amino terminus

Sites at the N-terminus of GFP were engineered into GFP by PCR. Initial studies attempted to preserve the native sequence as much as possible. As discussed earlier the positions chosen for phosphorylation were within the first 5 amino acids of GFP and encompassed all charged residues within this region. The sequence changes, relative fluorescence, relative rates of phosphorylation and % change in fluorescence upon phosphorylation are tabulated in Table III.

**Table III: Relative fluorescence, rate of phosphorylation and change in fluorescence upon phosphorylation for phosphorylation sites inserted at the N-terminus**

| SEQ ID NO: | Sequence | Relative fluorescence as a % of wild type | Relative rates of phosphorylation | % Change in fluorescence |
|---|---|---|---|---|
| 2 | 1MSKGEELF | 100 | 1.0 | 0 |
| 25 | 1MRKGSCLF | 40 | 5.1 | 5.7 |
| 26 | 1MRKGSLLF | 52 | 1.6 | 8.0 |
| 27 | 1MRRESLLF | 30 | 3.0 | 6.0 |
| 28 | 1MRRDSCLF | 27 | 3.7 | 17 |
| 29 | 1MSRRDSCF | 43 | 2.1 | 25 |
| 30 | IMSKRRDSL | 7 | 5.5 | 5.1 |

Numbers prior to the sequence indicate amino acid position in wild type GFP where phosphorylation site starts. The relative rates of phosphorylation compare the rate of phosphorylation of the given phosphorylation site with the endogenous protein kinase A phosphorylation site in *Aequorea* GFP (HKFSV) measured by incorporation of ³²P after incubation of the purified substrate and protein kinase A catalytic subunit in the presence of ³²P-labelled ATP using the standard protocols described earlier. The % change in fluorescence represents the change in fluorescence (488 nm excitation, 511 nm emission) observed in each purified protein as a result of incubation with excess protein kinase A catalytic subunit for 1 hour at 30° C using phosphorylation conditions described earlier.

These results demonstrated that mutants whose sequence closely resembles the native protein retain considerable fluorescence, display good kinetics of phosphorylation, but show relatively small changes in fluorescence after phosphorylation. To improve the effect of phosphorylation on fluorescence, amino acids around the phosphorylation site were mutated to create an optimal phosphorylation sequence even if it disordered the existing local tertiary structure. Such disruption was predicted and found to decrease the basal fluorescence of these constructs in their non-phosphorylated state (Table IV).

**Table IV: Relative fluorescence before phosphorylation and change in fluorescence upon phosphorylation for more drastically altered phosphorylation sites inserted at the N-terminus**

| SEQ ID NO: | Sequence | Relative fluorescence, as a % of wild-type | % Change in fluorescence upon phosphorylation |
|---|---|---|---|
| 2 | 1MSKGEELF (=WT) | ≡100 | 0 |
| 31 | 1MSRRRRSI | 5.8 | 40 |
| 32 | 1MRRRRSII | 5.1 | 70 |
| 33 | -1MRRRRSIII | n.d. | 43 |
| 34 | -2MRRRRSIIIF | 0.7 | 15 |
| 35 | -3MRRRRSIIIIF | 0.6 | 70 |

Numbers prior to the sequence indicate amino acid position in wild type GFP where phosphorylation site starts. Negative numbers indicate extensions onto the wild-type N-terminus. The % change in fluorescence represents the change in fluorescence (488 excitation, 511 emission) observed in each purified protein resulting from incubation with excess protein kinase A catalytic subunit for 1 hour at 30° C using standard phosphorylation conditions described earlier.

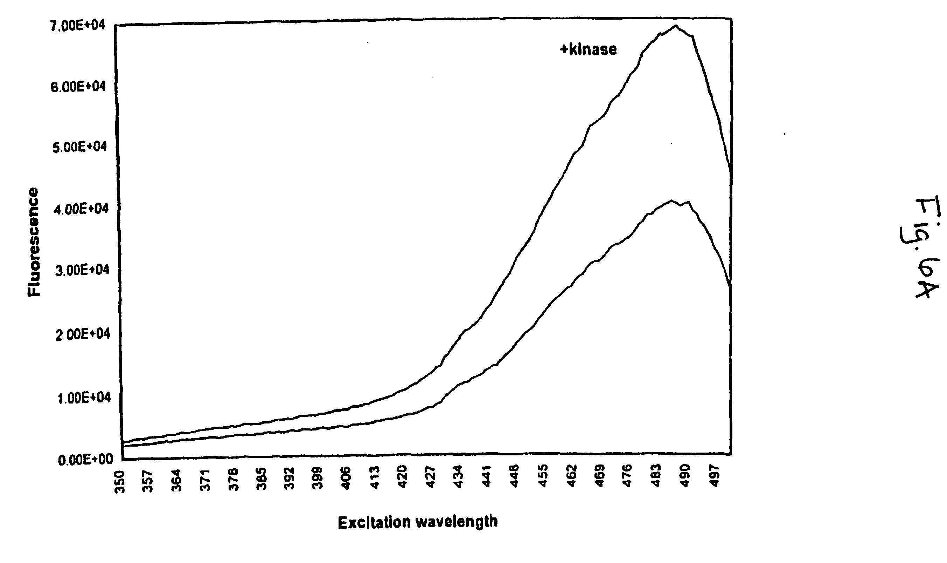

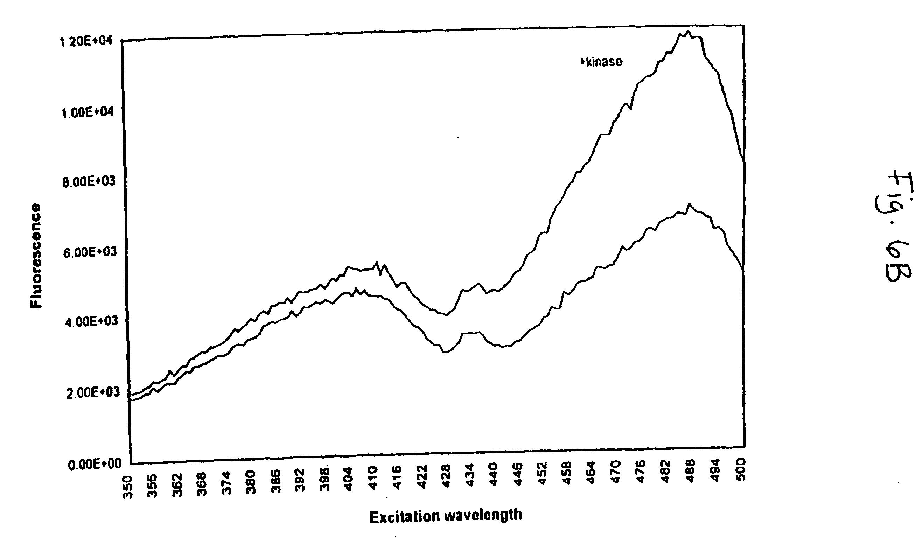

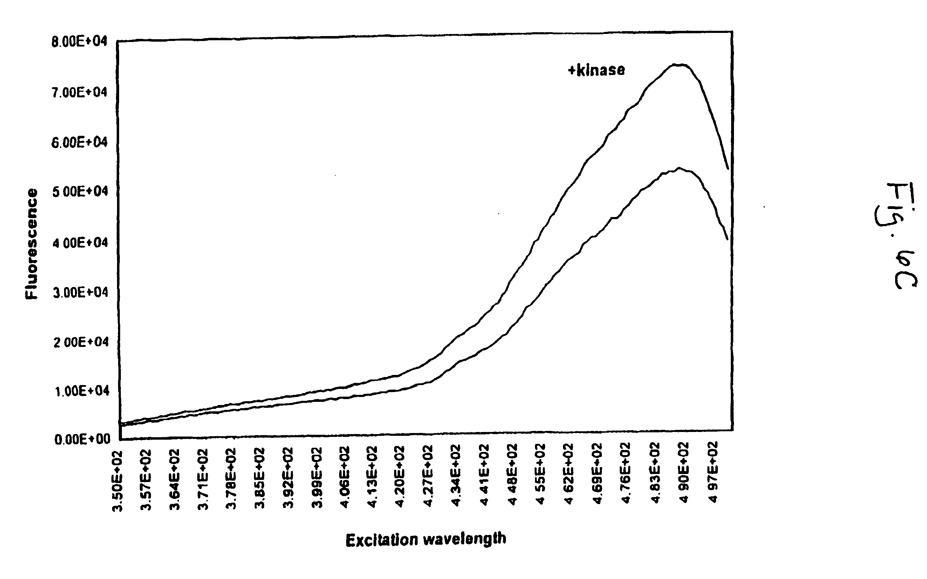

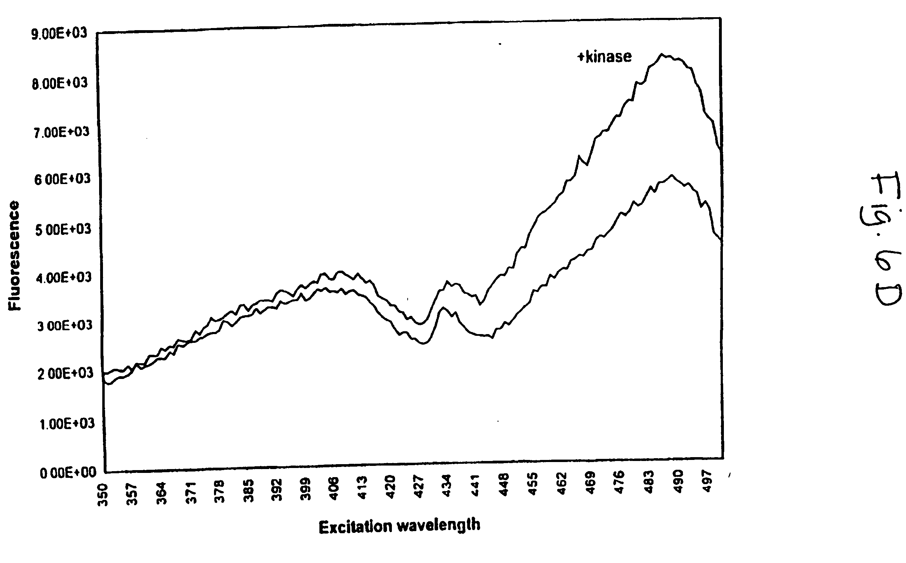

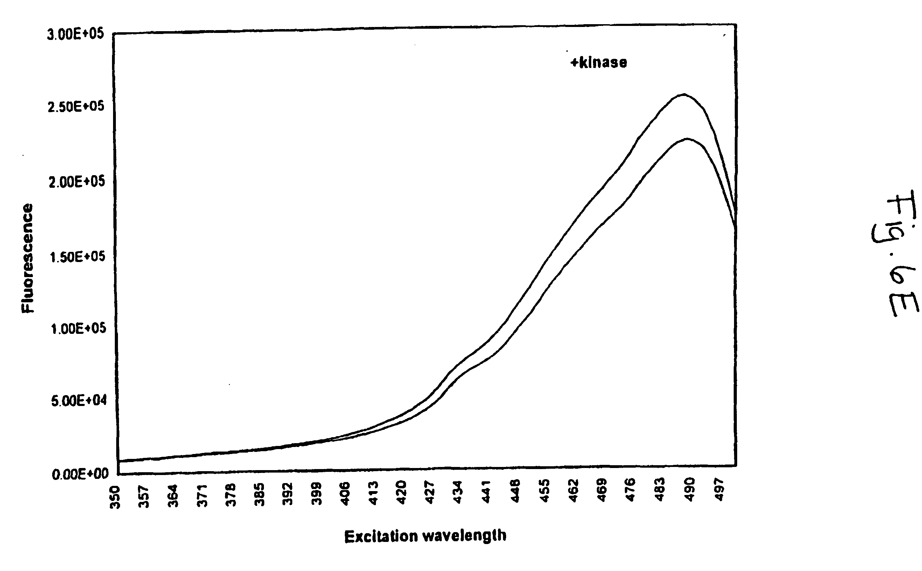

Perhaps because of the reduced basal fluorescence, phosphorylation by protein kinase A produced greater percentage increases in fluorescence in these constructs than in the more conservative mutations of Table II. Constructs 1MRRRRSII (SEQ ID NO:32) and -3MRRRRSIIIIF (SEQ ID NO:35) displayed the greatest increases, about 70%, in fluorescence upon phosphorylation using the standard conditions, as shown in Fig. 6. However, these increased percentage increases were obtained at the cost of a reduced ability to fold at higher temperatures and relatively poor fluorescence even after phosphorylation. To improve these characteristics, these mutants were further optimized by additional random mutagenesis with a novel selection procedure.

### C. Further optimization of N-terminal phosphorylation sites by random mutagenesis of the remainder of GFP

The two best constructs from above (1MRRRRSII (SEQ ID NO:32) and-3MRRRRSIII IF (SEQ ID NO:35)) were further mutagenized and screened for variants that are highly fluorescent when phosphorylated, but weakly fluorescent when non-phosphorylated. The method involved expression of a randomly mutated fluorescent substrate with or without simultaneous co-expression of the constitutively active catalytic subunit of protein kinase A in bacteria, and screening the individual mutants to determine those that are highly fluorescent in the presence but not the absence of the kinase.

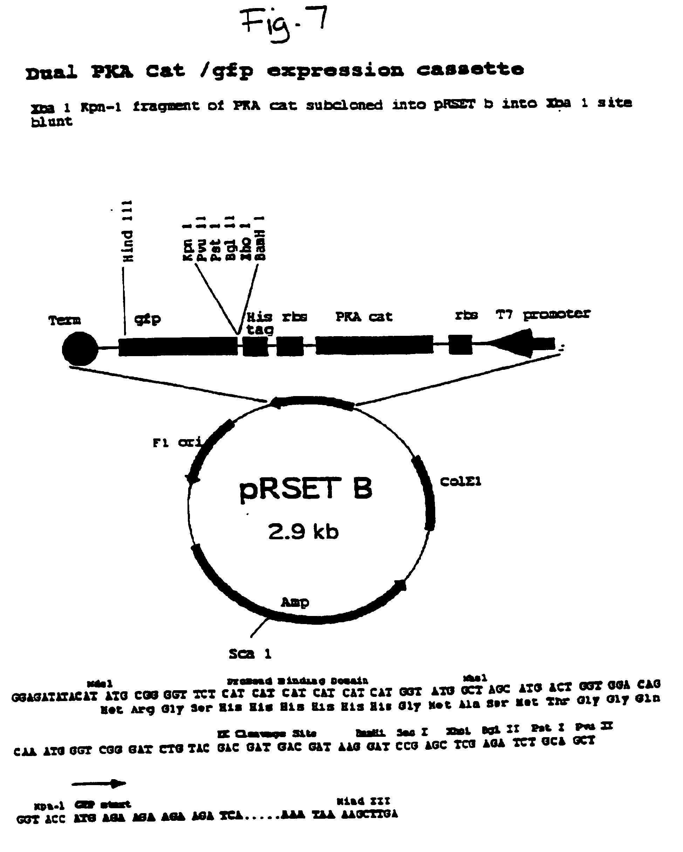

To enable co-expression of the kinase and potential substrates, a new expression vector with the kinase C subunit upstream from the fluorescent substrate was constructed (Fig. 7). Random mutations were introduced into GFP by error-prone PCR and the resulting population of mutants cloned into the co-expression vector using the appropriate restriction sites. The expression vector containing the mutated fluorescent substrates were transformed into host bacteria and individual bacterial colonies (each derived from a single cell, and hence containing a single unique mutant fluorescent substrate) were grown up.

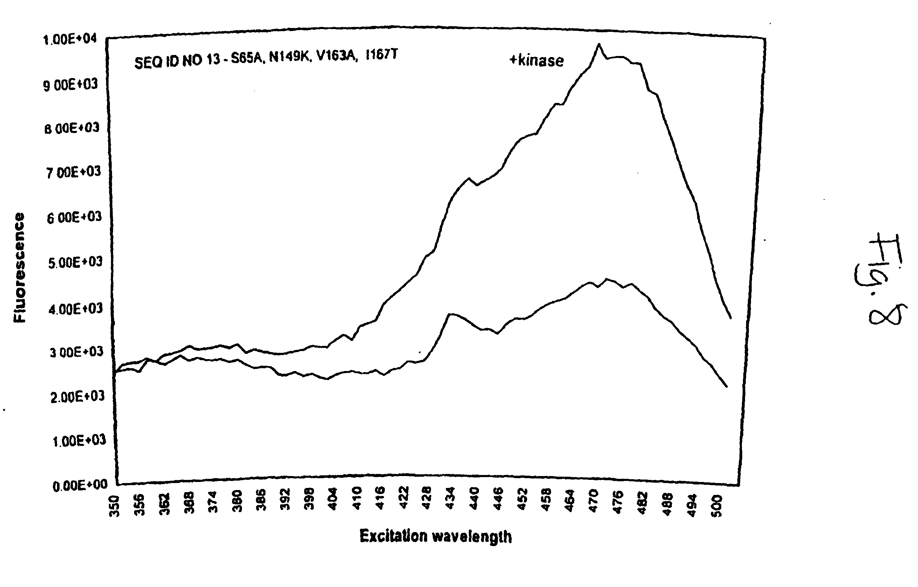

The colonies were screened for fluorescence either by fluorescence-activated cell sorting (Fig. 8) or by observation under a microscope. Those that exhibited the greatest fluorescence were re-screened under conditions in which the kinase gene was inactivated. This was achieved in either of two ways. In the first method the co-expression vector was isolated and treated with restriction endonucleases and modifying enzymes (EcoR1, klenow fragment and T4 DNA ligase) to cut the kinase gene, add additional bases and religate the DNA, causing a frame shift and hence inactivating the gene. The treated and non-treated plasmids were then re-transformed into bacteria and compared in fluorescence. Alternatively the plasmids were initially grown in a RecA (recombinase A negative) bacterial strain, where the kinase is stable, to screen for brighter mutants in the presence of the kinase. The plasmid DNA was then isolated and re-transformed into a strain of bacteria which is RecA⁺, in which the kinase is unstable and is lost through homologous recombination of the tandomly repeated ribosome binding sites (rbs). The bacteria have a strong tendency to eliminate the kinase C subunit because it slows their multiplication, so cells that splice out the kinase by recombination have a large growth advantage.

Comparison of the brightness of the mutant first in the presence of kinase then in its absence indicates the relative effect of phosphorylation on the mutant GFP fluorescence (after normalizing for GFP expression levels). A library of approximately 2 x10⁶ members was screened by this approach. Approximately 500 displayed higher levels of fluorescence when screened in the presence of the kinase. After inactivation of the kinase, one mutant out of the 500 displayed reduced levels of fluorescence. The increased fluorescence of the remainder of the 500 mutants was independent of the presence of the kinase. This mutant GFP was isolated and sequenced and found to contain the following mutations compared to wild-type GFP (Fig. 3, SEQ ID NO:2) (in addition to the N-terminal phosphorylation site 1MRRRRSII (SEQ ID NO:32)): S65A, N149K, V163A and I167T.

To confirm that this mutant was indeed directly sensitive to protein kinase A phosphorylation and to quantify its responsively, it was expressed in the absence of kinase. The *E. coli* were lysed and the protein purified as described earlier using a nickel affinity column. The protein exhibited high levels of fluorescence when induced at 30° C but displayed reduced fluorescence when incubated at 37°C. After such preincubation (37°C overnight) and separation of the less fluorescent material by centrifugation, this protein exhibited the largest change in fluorescence upon phosphorylation yet observed (Fig. 8). The tolerance of this mutant for 37°C treatment suggests that this mutant is suitable for use in mammalian cells.

The present invention provides novel assays for protein kinase activity involving novel fluorescent protein substrates. While specific examples have been provided, the above description is illustrative and not restrictive. Many variations of the invention will become apparent to those skilled in the art upon review of this specification. The scope of the invention should, therefore, be determined not with reference to the above description, but instead should be determined with reference to the appended claims along with their full scope of equivalents.

## Claims

1. A method for determining whether a sample contains protein kinase activity comprising:
contacting the sample with a phosphate donor and a fluorescent protein substrate for a protein kinase, the protein substrate comprising a fluorescent protein with at least 85% sequence identity over any 150 contiguous amino acids with the amino acid sequence of SEQ ID NO:2, further containing a phosphorylation site selected from the group consisting of RRFSV at positions 25-29, RRFSA at positions 69-73, KRDSM at positions 214-218, MRKGSCLF at positions 1-8, MRKGSLLF at positions 1-8, MRRESLLF at positions 1-8, MRRDSCLF at positions 1-8, MSRRDSCF at positions 1-8, MSKRRDLS at positions 1-8, MSRRRRSI at position 1-8, MRRRRSII at positions 1-8, MRRRRSIII at positions -1-8, MRRRRSIIIF at positions -2-8, and MRRRRSIIIIF at positions-3-8, wherein the protein substrate exhibits a different fluorescent property in the phosphorylated state than in the un-phosphorylated state;
exciting the protein substrate; and
measuring the amount of a fluorescent property that differs in the unphosphorylated state and phosphorylated state, whereby an amount that is consistent with the presence of the protein substrate in its phosphorylated state indicates the presence of protein kinase activity.

2. The method of claim 1 for determining the amount of protein kinase activity in a sample wherein measuring the amount of a fluorescent property in the sample comprises measuring the amount at two or more time points after contacting the sample with a phosphate donor and a fluorescent protein substrate, and determining the quantity of change or rate of change of the measured amount, whereby the quantity or rate of change of the measured amount reflects the amount of protein kinase activity in the sample.

3. An in vitro method for determining whether a cell exhibits protein kinase activity comprising the steps of:
providing a transfected host cell comprising a recombinant nucleic acid molecule comprising expression control sequences operatively linked to a nucleic acid sequence coding for the expression of a fluorescent protein substrate for a protein kinase, the protein substrate comprising a fluorescent protein with at least 85% sequence identity over any 150 contiguous amino acids of SEQ ID NO:2, further containing a phosphorylation site selected from the group consisting of RRFSV at positions 25-29, RRFSA at positions 69-73, KRDSM at positions 214-218, MRKGSCLF at positions 1-8, MRKGSLLF at positions 1-8, MRRESLLF at positions 1-8, MRRDSCLF at positions 1-8, MSRRDSCF at positions 1-8, MSKRRDLS at positions 1-8, MSRRRRSI at position 1-8, MRRRRSII at positions 1-8, MRRRRSIII at positions -1-8, MRRRRSIIIF at positions -2-8, and MRRRRSIIIIF at positions -3-8, wherein the protein substrate exhibits a different fluorescent property in the phosphorylated state than in the un-phosphorylated state, the cell expressing the fluorescent protein substrate;
exciting the protein substrate in the cell; and
measuring the amount of a fluorescent property that differs in the unphosphorylated and phosphorylated states, wherein the presence of the fluorescent property associated with the fluorescent state indicates the presence of protein kinase activity in the cell.

4. The method of claim 3 wherein the cell is further transfected with an expression vector comprising expression control sequences operatively linked to a nucleic acid sequence coding for the expression of the protein kinase.

5. The method of claim 3 or 4 wherein the step of providing a transfected host cell comprises inducing expression of the protein substrate to produce a sudden increase in the expression of the protein substrate, and the step of measuring the amount of a fluorescent property comprises measuring the amount at a first and a second time after expression of the protein substrate and determining the difference between the measured amounts at the first and second time.

6. An in vitro method for determining the amount of activity of a protein kinase in a sample from an organism comprising the steps of:
providing a sample from an organism having a cell that expresses a fluorescent protein substrate for a protein kinase, the protein substrate comprising a fluorescent protein with at least 85% sequence identity over any 150 contiguous amino acids of SEQ ID NO:2, further containing a phosphorylation site selected from the group consisting of RRFSV at positions 25-29, RRFSA at positions 69-73, KRDSM at positions 214-218, MRKGSCLF at positions 1-8, MRKGSLLF at positions 1-8, MRRESLLF at positions 1-8, MRRDSCLF at positions 1-8, MSRRDSCF at positions 1-8, MSKRRDLS at positions 1-8, MSRRRRSI at position 1-8, MRRRRSII at positions 1-8, MRRRRSIII at positions -1-8, MRRRRSIIIF at positions -2-8, and MRRRRSIIIIF at positions -3-8, wherein the protein substrate exhibits a different fluorescent property in the phosphorylated state than in the un-phosphorylated state;
contacting the sample with a phosphate donor;
exciting the protein substrate; and
measuring the amount of a fluorescent property that differs in the unphosphorylated state and phosphorylated state, whereby an amount that is consistent with the presence of the protein substrate in its phosphorylated state indicates the presence of protein kinase activity, and an amount that is consistent with the presence of the protein substrate in its un-phosphorylated state indicates the absence of protein kinase activity.

7. The method of claim 6 wherein the sample is a cell homogenate.

8. A method for determining whether a compound alters the activity of a protein kinase comprising the steps of:
contacting a sample containing a known amount of protein kinase activity with the compound, a phosphate donor for the protein kinase and a fluorescent protein substrate for a protein kinase, the protein substrate comprising a fluorescent protein with at least 85% sequence identity over any 150 contiguous amino acids of SEQ ID NO:2, further containing, a phosphorylation site selected from the group consisting of RRFSV at positions 25-29, RRFSA at positions 69-73, KRDSM at positions 214-218, MRKGSCLF at positions 1-8. MRKGSLLF at positions 1-8, MRRESLLF at positions 1-8, MRRDSCLF at positions 1-8. MSRRDSCF at positions 1-8. MSKRRDLS at positions 1-8, MSRRRRSI at position 1-8, MRRRRSIII at positions 1-8, MRRRRSIII at positions -1-8, MRRRRSIIIF at positions -2-8, and MRRRRSIIIIF at positions -3-8, wherein the protein substrate exhibits a different fluorescent property in the phosphorylated state than in the un-phosphorylated state; exciting the protein substrate;
measuring the amount of protein kinase activity in the sample as a function of the quantity of change or rate of change of a fluorescent property that differs in the un-phosphorylated and phosphorylated states; and
comparing the amount of activity in the sample with a standard activity for the same amount of the protein kinase, whereby a difference between the amount of protein kinase activity in the sample and the standard activity indicates that the compound alters the activity of the protein kinase.

9. A method for determining whether a compound alters the protein kinase activity in a cell comprising the steps of:
providing first and second transfected host cells exhibiting protein kinase activity and expressing a fluorescent protein substrate for a protein kinase, the protein substrate comprising a fluorescent protein with at least 85% sequence identity over any 150 contiguous amino acids of SEQ ID NO:2, further containing a phosphorylation site selected from the group consisting of RRFSV at positions 25-29, RRFSA at positions 69-73, KRDSM at positions 214-218, MRKGSCLF at positions 1-8, MRKGSCLF at positions 1-8, MRRESLLF at positions 1-8, MRRDSCLF at positions 1-8, MSRRDSCF at positions 1-8, MSKRRDLS at positions 1-8, MSRRRRSI at position 1-8. MRRRRSII at positions 1-8, MRRRRSIII at positions -1-8, MRRRRSIIIF at positions -2-8, and MRRRRSIIIIF at positions -3-8, wherein the protein substrate exhibits a different fluorescent property in the phosphorylated state than in the unphosphorylated state;
contacting the first cell with an amount of the compound;
contacting the second cell with a different amount of the compound;
exciting the protein substrate in the first and second cells;
measuring the amount of protein kinase activity in the cells as a function of the quantity of change or rate of change of a fluorescent property that differs in the un-phosphorylated and phosphorylated states in the first and second cells; and
comparing the amount in the first and second cells, whereby a difference in the amount indicates that the compound alters protein kinase activity in the cell.

10. The method of claim 9 wherein the cells are transfected with an expression vector comprising expression control sequences operatively linked to a nucleic acid sequence coding for the expression of the protein kinase.

11. The method of any one of claims 1 to 10, wherein the fluorescent property is the fluorescent emission around the emission maximum of the substrate in the phosphorylated state.

12. The method of any one of claims 1 to 11, wherein the amount of protein kinase activity is determined by emission amplitude ratioing or excitation amplitude ratioing.

13. A fluorescent protein substrate for a protein kinase comprising a fluorescent protein with at least 85% sequence identity over any 150 contiguous amino acids of SEQ ID NO:2, further containing a phosphorylation site selected from the group consisting of RRFSV at positions 25-29, RRFSA at positions 69-73, KRDSM at positions 214-218, MRKGSCLF at positions 1-8, MRKGSLLF at positions 1-8, MRRESLLF at positions 1-8, MRRDSCLF at positions 1-8, MSRRDSCF at positions 1-8, MSKRRDLS at positions 1-8, MSRRRRSI at position 1-8, MRRRRSII at positions 1-8, MRRRRSIII at positions -1-8, MRRRRSIIIF at positions -2-8, and MRRRRSIIIIF at positions -3-8, wherein the protein substrate exhibits a different fluorescent property in the phosphorylated state than in the un-phosphorylated state.

14. The protein substrate of claim 13, wherein the fluorescent protein is variant P4, P4-3, W7, W2, S65T, P4-1, S65A, S65L, Y66F, or Y66W.

15. The protein substrate of either of claims 13 or 14, wherein the fluorescent protein further comprises a folding mutation selected from T44A, I64L, V68L, S724, I99S, Y145I, N146I, M153(T or A), V163A, I167T, S175G, S205T, and N212K.

16. The protein substrate of any one of claims 13 to 15, further comprising the substitutions S65A, N149K, V163A, and I167T.

17. The protein substrate of any one of claims 13 to 16 further comprising the substitution H217S.

18. The protein substrate of any one of claims 13 to 17 further comprising the substitution E171K and/or I172V.

19. The protein substrate of any one of claims 13 to 18 wherein the protein substrate is a fusion protein.

20. A nucleic acid molecule coding for the expression of a fluorescent protein substrate as defined in any one of claims 13 to 19.

21. A recombinant nucleic acid molecule comprising expression control sequences operatively linked to a nucleic acid sequence coding for the expression of a fluorescent protein substrate for a protein kinase comprising a fluorescent protein with at least 85% sequence identity over any 150 contiguous amino acids of SEQ ID NO:2, further containing a phosphorylation site selected from the group consisting of RRFSV at positions 25-29, RRFSA at positions 69-73, KRDSM at positions 214-218, MRKGSCLF at positions 1-8, MRKGSLLF at positions 1-8, MRRESLLF at positions 1-8, MRRDSCLF at positions 1-8, MSRRDSCF at positions 1-8, MSKRRDLS at positions 1-8, MSRRRRSI at position 1-8, MRRRRSII at positions 1-8, MRRRRSIII at positions -1-8, MRRRRSIIIF at positions -2-8, and MRRRRSIIIIF at positions -3-8, wherein the protein substrate exhibits a different fluorescent property in the phosphorylated state than in the un-phosphorylated state.

22. A transfected host cell comprising a recombinant nucleic acid molecule comprising expression control sequences operatively linked to a nucleic acid sequence coding for the expression of a fluorescent protein substrate for a protein kinase comprising a fluorescent protein with at least 85% sequence identity over any 150 contiguous amino acids of SEQ ID NO:2, further containing a phosphorylation site selected from the group consisting of RRFSV at positions 25-29, RRFSA at positions 69-73, KRDSM at positions 214-218, MRKGSCLF at positions 1-8, MRKGSLLF at positions 1-8, MRRESLLF at positions 1-8, MRRDSCLF at positions 1-8, MSRRDSCF at positions 1-8, MSKRRDLS at positions 1-8, MSRRRRSI at position 1-8, MRRRRSII at positions 1-8, MRRRRSIII at positions -1-8, MRRRRSIIIF at positions -2-8, and MRRRRSIIIIF at positions -3-8, wherein the protein substrate exhibits a different fluorescent property in the phosphorylated state than in the unphosphorylated state.

23. An in vitro method for screening a collection of transfected host cells comprising:
providing a collection of transfected host cells comprising at least 10 different host cell members, each member expressing a different fluorescent protein candidate substrate, the candidate substrate comprising a fluorescent protein with at least 85% sequence identity over any 150 contiguous amino acids with the amino acid sequence of SEQ ID NO:2 and a variable peptide moiety within about twenty amino acids of the amino-terminus of the fluorescent protein moiety, wherein the fluorescent protein exhibits a fluorescent property;
measuring the fluorescent property in a sample comprising at least one host cell member before and after increasing or decreasing the intracellular protein kinase activity in the cell; and
determining the degree of change or the rate of change in the fluorescent property
upon increasing or decreasing intracellular protein kinase activity;
whereby a change in the degree or rate of the fluorescent property indicates that the candidate substrate possesses a peptide moiety that can be phosphorylated by the protein kinase and whose phosphorylation alters the fluorescent property.

24. The method of claim 23 wherein the variable peptide moiety is within about 5 amino acids of the amino-terminus.

25. The method of claim 23 further comprising determining the change in a plurality of host cell members and comparing the degree of change or rate of change between the host cell members; whereby the comparison indicates in the candidate substrates the relative change in the fluorescent property upon phosphorylation.

26. The method of any one of claims 23 to 25 wherein the host cell is co-transfected with an expression vector that expresses a protein kinase comprising expression control sequences operatively linked to a sequence that codes for the expression of the protein kinase.

27. The method of claim 26 wherein the step of increasing the intracellular protein kinase activity comprises elevating intracellular cAMP to activate cAMP-dependent protein kinase, elevating intracellular cGMP to activate cGMP-dependent protein kinase, elevating cytosolic free calcium to activate Ca2+/calmodulin-dependent protein kinase types I, II, or IV, or administration of phorbol myristate acetate to activate protein kinase C.

28. The method of any one of claims 23 to 26 wherein the step of decreasing the intracellular protein kinase activity comprises culturing the cell for a time sufficient for the cell to lose sequence that codes for the expression of the protein kinase.

29. The method of any one of claims 23 to 28 further comprising isolating the recombinant nucleic acid molecule coding for the expression of the candidate substrate.

30. An in vitro method for screening a collection of transfected host cells comprising:
providing a lysate from each of at least one member of a collection of transfected host cells comprising at least 10 different host cell members, each member expressing a different fluorescent protein candidate substrate, the candidate substrate comprising a fluorescent protein with at least 85% sequence identity over any 150 contiguous amino acids with the amino acid sequence of SEQ ID NO:2 and a variable peptide moiety around the amino-terminus of the fluorescent protein moiety, wherein the fluorescent protein exhibits a fluorescent property; contacting the lysate with a phosphate donor;
measuring the fluorescent property from at least one lysate before and after contacting the lysate with a protein kinase; and
determining the degree of change or the rate of change in the fluorescent property upon contacting the lysate with intracellular protein kinase activity;
whereby a change in the degree or rate of the fluorescent property indicates that the candidate substrate possesses a peptide moiety that can be phosphorylated by the protein kinase and whose phosphorylation alters the fluorescent property.

31. The method of claim 30 comprising splitting the lysate into first and second aliquots;
contacting the first and second aliquots with a phosphate donor;
contacting the first aliquot with a protein kinase but not contacting the second aliquot with the protein kinase;
measuring the fluorescent property from the first aliquot before and after contacting the lysate with the protein kinase;
measuring the fluorescent property from the second aliquot at a plurality of time points after contact with the phosphate donor;
determining the degree of change or the rate of change in the fluorescent property of the first and second aliquots;
comparing the degree or rate of change in the first and second aliquots;
whereby a difference in the degree or rate of change in the fluorescent property in first and second aliquots indicates that the candidate substrate possesses a peptide moiety that alters the fluorescent property.

32. The method of claim 30 or 31 wherein the amount is determined by emission amplitude ratioing or excitation amplitude ratioing.

33. The method of claim 25 further comprising isolating a member having an altered fluorescent property.

34. The method of any one of claims 23 to 33 wherein the step of determining the rate of change comprises measuring the fluorescent property at a plurality of time points after inducing intracellular protein kinase activity.

35. The method of any one of claims 23 to 34 wherein the host cell is expanded into a clonal population.

## Patentansprüche

1. Verfahren zur Bestimmung, ob eine Probe Proteinkinaseaktivität enthält, umfassend:
das Inkontaktbringen der Probe mit einem Phosphatdonor und einem Fluoreszenzprotein-Substrat für eine Proteinkinase, wobei das Proteinsubstrat ein Fluoreszenzprotein umfasst mit mindestens 85% Sequenzidentität über beliebige 150 zusammenhängende Aminosäuren mit der Aminosäuresequenz von SEQ ID NO:2, ferner enthaltend eine Phosphorylierungsstelle ausgewählt aus der Gruppe bestehend aus RRFSV an den Positionen 25-29, RRFSA an den Positionen 69-73, KRDSM an den Positionen 214-218, MRKGSCLF an den Positionen 1-8, MRKGSLLF an den Positionen 1-8, MRRESLLF an den Positionen 1-8, MRRDSCLF an den Positionen 1-8, MSRRDSCF an den Positionen 1-8, MSKRRDLS an den Positionen 1-8, MSRRRRSI an den Positionen 1-8, MRRRRSII an den Positionen 1-8, MRRRRSIII an den Positionen -1-8, MRRRRSIIIF an den Positionen -2-8 und MRRRRSIIIIF an den Positionen -3-8, wobei das Proteinsubstrat eine andere Fluoreszenzeigenschaft im phosphorylierten als im unphosphorylierten Zustand aufweist;
das Anregen des Proteinsubstrats; und
das Messen der Menge einer Fluoreszenzeigenschaft, die sich im unphosphorylierten Zustand und dem phosphorylierten Zustand unterscheidet,
wobei eine Menge, die einhergeht mit dem Vorliegen des Proteinsubstrats in seinem phosphorylierten Zustand das Vorliegen einer Proteinkinaseaktivität anzeigt.

2. Verfahren nach Anspruch 1 zur Bestimmung der Menge einer Proteinkinaseaktivität in einer Probe, wobei das Messen der Menge einer Fluoreszenzeigenschaft in der Probe das Messen der Menge zu zwei oder mehr Zeitpunkten nach Inkontaktbringen der Probe mit einem Phosphatdonor und einem Fluoreszenzprotein-Substrat umfasst, und das Bestimmen des Umfangs der Veränderung oder der Veränderungsrate der gemessenen Menge, wobei der Umfang der Veränderung oder die Veränderungsrate der gemessenen Menge die Menge an Proteinkinaseaktivität in der Probe widerspiegelt.

3. In vitro-Verfahren zur Bestimmung, ob eine Zelle Proteinkinaseaktivität aufweist, umfassend die Schritte:
das Bereitstellen einer transfizierten Wirtszelle umfassend ein rekombinantes Nucleinsäuremolekül umfassend Expressionskontrollsequenzen, die funktionell verknüpft sind mit einer Nucleinsäuresequenz, die die Expression eines Fluoreszenzprotein-Substrats für eine Proteinkinase codiert, wobei das Proteinsubstrat ein Fluoreszenzprotein umfasst mit mindestens 85% Sequenzidentität über beliebige 150 zusammenhängende Aminosäuren von SEQ ID NO:2, ferner enthaltend eine Phosphorylierungsstelle ausgewählt aus der Gruppe bestehend aus RRFSV an den Positionen 25-29, RRFSA an den Positionen 69-73, KRDSM an den Positionen 214-218, MRKGSCLF an den Positionen 1-8, MRKGSLLF an den Positionen 1-8, MRRESLLF an den Positionen 1-8, MRRDSCLF an den Positionen 1-8, MSRRDSCF an den Positionen 1-8, MSKRRDLS an den Positionen 1-8, MSRRRRSI an den Positionen 1-8, MRRRRSII an den Positionen 1-8, MRRRRSIII an den Positionen -1-8, MRRRRSIIIF an den Positionen -2-8 und MRRRRSIIIIF an den Positionen -3-8, wobei das Proteinsubstrat eine andere Fluoreszenzeigenschaft im phosphorylierten als im unphosphorylierten Zustand aufweist, wobei die Zelle das Fluoreszenzprotein-Substrat exprimiert;
das Anregen des Proteinsubstrats in der Zelle; und
das Messen der Menge einer Fluoreszenzeigenschaft, die sich im unphosphorylierten Zustand und dem phosphorylierten Zustand unterscheidet,
wobei das Vorliegen der Fluoreszenzeigenschaft in Assoziation mit dem Fluoreszenzzustand das Vorliegen einer Proteinkinaseaktivität in der Zelle anzeigt.

4. Verfahren nach Anspruch 3, wobei die Zelle ferner transfiziert ist mit einem Expressionsvektor, der Expressionskontrollsequenzen umfasst, die funktionell verknüpft sind mit einer Nucleinsäuresequenz, die die Expression der Proteinkinase codiert.

5. Verfahren nach Anspruch 3 oder 4, wobei der Schritt des Bereitstellens einer transfizierten Wirtszelle das Induzieren der Expression des Proteinsubstrats umfasst, um eine unvermittelte Erhöhung der Expression des Proteinsubstrats zu erzeugen, und der Schritt des Messens der Menge einer Fluoreszenzeigenschaft das Messen der Menge zu einem ersten und einem zweiten Zeitpunkt nach der Expression des Proteinsubstrats und das Bestimmen des Unterschieds zwischen der gemessenen Menge beim ersten und zweiten Zeitpunkt umfasst.

6. In vitro-Verfahren zur Bestimmung der Menge einer Proteinkinaseaktivität in einer Probe von einem Organismus umfassend die Schritte:
das Bereitstellen einer Probe von einem Organismus, der eine Zelle hat, die ein Fluoreszenzprotein-Substrat für eine Proteinkinase exprimiert, wobei das Proteinsubstrat ein Fluoreszenzprotein umfasst mit mindestens 85% Sequenzidentität über beliebige 150 zusammenhängende Aminosäuren von SEQ ID NO:2, ferner enthaltend eine Phosphorylierungsstelle ausgewählt aus der Gruppe bestehend aus RRFSV an den Positionen 25-29, RRFSA an den Positionen 69-73, KRDSM an den Positionen 214-218, MRKGSCLF an den Positionen 1-8, MRKGSLLF an den Positionen 1-8, MRRESLLF an den Positionen 1-8, MRRDSCLF an den Positionen 1-8, MSRRDSCF an den Positionen 1-8, MSKRRDLS an den Positionen 1-8, MSRRRRSI an den Positionen 1-8, MRRRRSII an den Positionen 1-8, MRRRRSIII an den Positionen -1-8, MRRRRSIIIF an den Positionen -2-8 und MRRRRSIIIIF an den Positionen -3-8, wobei das Proteinsubstrat eine andere Fluoreszenzeigenschaft im phosphorylierten als im unphosphorylierten Zustand aufweist;
das Inkontaktbringen der Probe mit einem Phosphatdonor;
das Anregen des Proteinsubstrats; und
das Messen der Menge einer Fluoreszenzeigenschaft, die sich im unphosphorylierten Zustand und dem phosphorylierten Zustand unterscheidet,
wobei eine Menge, die einhergeht mit dem Vorliegen des Proteinsubstrats in seinem phosphorylierten Zustand, das Vorliegen einer Proteinkinaseaktivität anzeigt, und eine Menge, die einhergeht mit dem Vorliegen des Proteinsubstrats in seinem unphosphorylierten Zustand, das Fehlen einer Proteinkinaseaktivität anzeigt.

7. Verfahren nach Anspruch 6, wobei die Probe ein Zellhomogenat ist.

8. Verfahren zur Bestimmung, ob eine Verbindung die Aktivität einer Proteinkinase verändert, umfassend die Schritte:
das Inkontaktbringen einer Probe, die eine bekannte Menge einer Proteinkinaseaktivität enthält, mit der Verbindung, einem Phosphatdonor für die Proteinkinase und einem Fluoreszenzprotein-Substrat für eine Proteinkinase, wobei das Proteinsubstrat ein Fluoreszenzprotein umfasst mit mindestens 85% Sequenzidentität über beliebige 150 zusammenhängende Aminosäuren von SEQ ID NO:2, ferner enthaltend eine Phosphorylierungsstelle ausgewählt aus der Gruppe bestehend aus RRFSV an den Positionen 25-29, RRFSA an den Positionen 69-73, KRDSM an den Positionen 214-218, MRKGSCLF an den Positionen 1-8, MRKGSLLF an den Positionen 1-8, MRRESLLF an den Positionen 1-8, MRRDSCLF an den Positionen 1-8, MSRRDSCF an den Positionen 1-8, MSKRRDLS an den Positionen 1-8, MSRRRRSI an den Positionen 1-8, MRRRRSII an den Positionen 1-8, MRRRRSIII an den Positionen -1-8, MRRRRSIIIF an den Positionen -2-8 und MRRRRSIIIIF an den Positionen -3-8, wobei das Proteinsubstrat eine andere Fluoreszenzeigenschaft im phosphorylierten als im unphosphorylierten Zustand aufweist;
das Anregen des Proteinsubstrats; und
das Messen der Menge der Proteinkinaseaktivität in der Probe als eine Funktion des Umfangs der Veränderung oder der Veränderungsrate einer Fluoreszenzeigenschaft, die sich im unphosphorylierten Zustand und dem phosphorylierten Zustand unterscheidet; und
das Vergleichen der Menge an Aktivität in der Probe mit einer Standardaktivität derselben Menge der Proteinkinase, wobei ein Unterschied zwischen der Menge der Proteinkinaseaktivität in der Probe und der Standardaktivität anzeigt, dass die Verbindung die Aktivität der Proteinkinase verändert.

9. Verfahren zur Bestimmung, ob eine Verbindung die Proteinkinaseaktivität in einer Zelle verändert, umfassend die Schritte:
das Bereitstellen einer ersten und zweiten transfizierten Wirtszelle, die eine Proteinkinaseaktivität aufweisen und ein Fluoreszenzprotein-Substrat für eine Proteinkinase exprimieren, wobei das Proteinsubstrat ein Fluoreszenzprotein umfasst mit mindestens 85% Sequenzidentität über beliebige 150 zusammenhängende Aminosäuren von SEQ ID NO:2, ferner enthaltend eine Phosphorylierungsstelle ausgewählt aus der Gruppe bestehend aus RRFSV an den Positionen 25-29, RRFSA an den Positionen 69-73, KRDSM an den Positionen 214-218, MRKGSCLF an den Positionen 1-8, MRKGSLLF an den Positionen 1-8, MRRESLLF an den Positionen 1-8, MRRDSCLF an den Positionen 1-8, MSRRDSCF an den Positionen 1-8, MSKRRDLS an den Positionen 1-8, MSRRRRSI an den Positionen 1-8, MRRRRSII an den Positionen 1-8, MRRRRSIII an den Positionen -1-8, MRRRRSIIIF an den Positionen -2-8 und MRRRRSIIIIF an den Positionen -3-8, wobei das Proteinsubstrat eine andere Fluoreszenzeigenschaft im phosphorylierten als im unphosphorylierten Zustand aufweist;
das Inkontaktbringen der ersten Zelle mit einer Menge der Verbindung;
das Inkontaktbringen der zweiten Zelle mit einer unterschiedlichen Menge der Verbindung;
das Anregen des Proteinsubstrats in der ersten und zweiten Zelle;
das Messen der Menge der Proteinkinaseaktivität in den Zellen als Funktion des Umfangs der Veränderung oder der Veränderungsrate einer Fluoreszenzeigenschaft, die sich im unphosphorylierten Zustand und dem phosphorylierten Zustand in der ersten und zweiten Zelle unterscheidet; und
das Vergleichen der Menge in der ersten und zweiten Zelle, wobei ein Unterschied in der Menge anzeigt, dass die Verbindung die Aktivität der Proteinkinase in der Zelle verändert.

10. Verfahren nach Anspruch 9, wobei die Zellen mit einem Expressionsvektor transfiziert sind, der Expressionskontrollsequenzen umfasst, die funktionell verknüpft sind mit einer Nucleinsäuresequenz, die die Expression der Proteinkinase codiert.

11. Verfahren nach einem der Ansprüche 1 bis 10, wobei die Fluoreszenzeigenschaft die Fluoreszenzemission um das Emissionsmaximum des Substrats im phosphorylierten Zustand ist.

12. Verfahren nach einem der Ansprüche 1 bis 11, wobei die Menge an Proteinkinaseaktivität bestimmt wird durch das Ins-Verhältnissetzen der Emissionsamplitude oder das Ins-Verhältnissetzen der Anregungsamplitude.

13. Fluoreszenzprotein-Substrat für eine Proteinkinase, umfassend ein Fluoreszenzprotein mit mindestens 85% Sequenzidentität über beliebige 150 zusammenhängende Aminosäuren von SEQ ID NO:2, ferner enthaltend eine Phosphorylierungsstelle ausgewählt aus der Gruppe bestehend aus RRFSV an den Positionen 25-29, RRFSA an den Positionen 69-73, KRDSM an den Positionen 214-218, MRKGSCLF an den Positionen 1-8, MRKGSLLF an den Positionen 1-8, MRRESLLF an den Positionen 1-8, MRRDSCLF an den Positionen 1-8, MSRRDSCF an den Positionen 1-8, MSKRRDLS an den Positionen 1-8, MSRRRRSI an den Positionen 1-8, MRRRRSII an den Positionen 1-8, MRRRRSIII an den Positionen -1-8, MRRRRSIIIF an den Positionen -2-8 und MRRRRSIIIIF an den Positionen -3-8, wobei das Proteinsubstrat eine andere Fluoreszenzeigenschaft im phosphorylierten als im unphosphorylierten Zustand aufweist.

14. Proteinsubstrat nach Anspruch 13, wobei das Fluoreszenzprotein Variante P4, P4-3, W7, W2, S65T, P4-1, S65A, S65L, Y66F oder Y66W ist.

15. Proteinsubstrat nach Anspruch 13 oder 14, wobei das Fluoreszenzprotein ferner eine Faltungsmutation umfasst, ausgewählt aus T44A, I64L, V68L, S72A, I99S, Y145I, N146I, M153(T oder A), V163A, I167T, S175G, S205T und N212K.

16. Proteinsubstrat nach einem der Ansprüche 13 bis 15, ferner umfassend die Austausche S65A, N149K, V163A und I167T.

17. Proteinsubstrat nach einem der Ansprüche 13 bis 16, ferner umfassend den Austausch H217S.

18. Proteinsubstrat nach einem der Ansprüche 13 bis 17, ferner umfassend die Austausche E171K und/oder I172V.

19. Proteinsubstrat nach einem der Ansprüche 13 bis 18, wobei das Proteinsubstrat ein Fusionsprotein ist.

20. Nucleinsäuremolekül, das die Expression eines Fluoreszenzprotein-Substrats nach einem der Ansprüche 13 bis 19 codiert.

21. Rekombinantes Nucleinsäuremolekül, umfassend Expressionskontrollsequenzen, die funktionell verknüpft sind mit einer Nucleinsäuresequenz, die die Expression eines Fluoreszenzprotein-Substrats einer Proteinkinase codiert, umfassend ein Fluoreszenzprotein mit mindestens 85% Sequenzidentität über beliebige 150 zusammenhängende Aminosäuren von SEQ ID NO:2, ferner enthaltend eine Phosphorylierungsstelle ausgewählt aus der Gruppe bestehend aus RRFSV an den Positionen 25-29, RRFSA an den Positionen 69-73, KRDSM an den Positionen 214-218, MRKGSCLF an den Positionen 1-8, MRKGSLLF an den Positionen 1-8, MRRESLLF an den Positionen 1-8, MRRDSCLF an den Positionen 1-8, MSRRDSCF an den Positionen 1-8, MSKRRDLS an den Positionen 1-8, MSRRRRSI an den Positionen 1-8, MRRRRSII an den Positionen 1-8, MRRRRSIII an den Positionen -1-8, MRRRRSIIIF an den Positionen -2-8 und MRRRRSIIIIF an den Positionen -3-8, wobei das Proteinsubstrat eine andere Fluoreszenzeigenschaft im phosphorylierten als im unphosphorylierten Zustand aufweist.

22. Transfizierte Wirtszelle, umfassend ein rekombinantes Nucleinsäuremolekül, umfassend Expressionskontrollsequenzen, die funktionell verknüpft sind mit einer Nucleinsäuresequenz, die die Expression eines Fluoreszenzprotein-Substrats für eine Proteinkinase codiert, umfassend ein Fluoreszenzprotein mit mindestens 85% Sequenzidentität über beliebige 150 zusammenhängende Aminosäuren von SEQ ID NO:2, ferner enthaltend eine Phosphorylierungsstelle ausgewählt aus der Gruppe bestehend aus RRFSV an den Positionen 25-29, RRFSA an den Positionen 69-73, KRDSM an den Positionen 214-218, MRKGSCLF an den Positionen 1-8, MRKGSLLF an den Positionen 1-8, MRRESLLF an den Positionen 1-8, MRRDSCLF an den Positionen 1-8, MSRRDSCF an den Positionen 1-8, MSKRRDLS an den Positionen 1-8, MSRRRRSI an den Positionen 1-8, MRRRRSII an den Positionen 1-8, MRRRRSIII an den Positionen -1-8, MRRRRSIIIF an den Positionen -2-8 und MRRRRSIIIIF an den Positionen -3-8, wobei das Proteinsubstrat eine andere Fluoreszenzeigenschaft im phosphorylierten als im unphosphorylierten Zustand aufweist.

23. In vitro-Verfahren zum Durchsuchen einer Sammlung transfizierter Wirtszellen umfassend:
das Bereitstellen einer Sammlung transfizierter Wirtszellen umfassend mindestens 10 verschiedene Wirtszellmitglieder, wobei jedes Mitglied einen unterschiedlichen Fluoreszenzprotein-Substratkandidaten exprimiert, wobei der Substratkandidat ein Fluoreszenzprotein umfasst mit mindestens 85% Sequenzidentität über beliebige 150 zusammenhängende Aminosäuren mit der Aminosäuresequenz von SEQ ID NO:2 und eine variable Peptideinheit innerhalb von etwa zwanzig Aminosäuren des Aminoterminus der Fluoreszenzproteineinheit, wobei das Fluoreszenzprotein eine Fluoreszenzeigenschaft aufweist;
das Messen der Fluoreszenzeigenschaft in einer Probe umfassend mindestens ein Wirtszellmitglied vor und nach Zunahme oder Abnahme der intrazellulären Proteinkinaseaktivität in der Zelle; und
das Bestimmen des Grads der Veränderung oder der Veränderungsrate in der Fluoreszenzeigenschaft nach Zunahme oder Abnahme der intrazellulären Proteinkinaseaktivität;
wobei eine Veränderung des Grads oder der Rate der Fluoreszenzeigenschaft anzeigt, dass der Substratkandidat eine funktionelle Peptideinheit besitzt, die durch die Proteinkinase phosphoryliert werden kann, und deren Phosphorylierung die Fluoreszenzeigenschaft verändert.

24. Verfahren nach Anspruch 23, wobei die variable Peptideinheit innerhalb von etwa 5 Aminosäuren des Aminoterminus ist.

25. Verfahren nach Anspruch 23, ferner umfassend das Bestimmen der Veränderung in einer Vielzahl von Wirtszellmitgliedern und das Vergleichen des Grads der Veränderung oder der Rate der Veränderung zwischen den Wirtszellmitgliedern; wobei das Vergleichen die relative Veränderung in der Fluoreszenzeigenschaft nach Phosphorylierung in den Substratkandidaten anzeigt.

26. Verfahren nach einem der Ansprüche 23 bis 25, wobei die Wirtszelle cotransfiziert ist mit einem Expressionvektor, der eine Proteinkinase exprimiert, der Expressionskontrollsequenzen umfasst, die funktionell verknüpft sind mit einer Nucleinsäuresequenz, die die Expression der Proteinkinase codiert.

27. Verfahren nach Anspruch 26, wobei der Schritt des Erhöhens der intrazellulären Proteinkinaseaktivität das Anheben des intrazellulären cAMPs, um cAMP-abhängige Proteinkinase zu aktivieren, das Anheben des intrazellulären cGMPs, um cGMP-abhängige Proteinkinase zu aktivieren, das Anheben des cytosolischen freien Calciums, um Ca²⁺/Calmodulin-abhängige Proteinkinasetypen I, II oder IV zu aktivieren, oder die Verabreichung von Phorbolmyristatacetat, um Proteinkinase C zu aktivieren, umfasst.

28. Verfahren nach einem der Ansprüche 23 bis 26, wobei der Schritt des Absenkens der intrazellulären Proteinkinaseaktivität das Züchten der Zelle über eine Zeit umfasst, die der Zelle ausreicht, die Sequenz, die die Expression der Proteinkinase codiert, zu verlieren.

29. Verfahren nach einem der Ansprüche 23 bis 28, ferner umfassend das Isolieren des rekombinanten Nucleinsäuremoleküls, das die Expression des Substratkandidaten codiert.

30. In vitro-Verfahren zum Durchsuchen einer Sammlung transfizierter Wirtszellen umfassend:
das Bereitstellen eines Lysats von jeweils mindestens einem Mitglied der Sammlung transfizierter Wirtszellen umfassend mindestens 10 verschiedene Wirtszellmitglieder, wobei jedes Mitglied einen unterschiedlichen Fluoreszenzprotein-Substratkandidaten exprimiert, wobei der Substratkandidat ein Fluoreszenzprotein umfasst mit mindestens 85% Sequenzidentität über beliebige 150 zusammenhängende Aminosäuren mit der Aminosäuresequenz von SEQ ID NO:2, und eine variable Peptideinheit um den Aminoterminus der Fluoreszenzproteineinheit, wobei das Fluoreszenzprotein eine Fluoreszenzeigenschaft aufweist; das Inkontaktbringen des Lysats mit einem Phosphatdonor;
das Messen der Fluoreszenzeigenschaft von mindestens einem Lysat vor und
nach dem Inkontaktbringen des Lysats mit einer Proteinkinase; und
das Bestimmen des Grads der Veränderung oder der Veränderungsrate in der Fluoreszenzeigenschaft nach Inkontaktbrigen des Lysats mit einer intrazellulären Proteinkinaseaktivität;
wobei eine Veränderung des Grads oder der Rate der Fluoreszenzeigenschaft anzeigt, dass der Substratkandidat eine Peptideinheit besitzt, die durch die Proteinkinase phosphoryliert werden kann, und deren Phosphorylierung die Fluoreszenzeigenschaft verändert.

31. Verfahren nach Anspruch 30, umfassend das Aufteilen des Lysats in ein erstes und zweites Aliquot;
das Inkontaktbringen des ersten und zweiten Aliquots mit einem Phosphatdonor;
das Inkontaktbringen des ersten Aliquots mit einer Proteinkinase, aber nicht das Inkontaktbringen des zweiten Aliquots mit der Proteinkinase;
das Messen der Fluoreszenzeigenschaft des ersten Aliquots vor und nach Inkontaktbringen des Lysats mit der Proteinkinase;
das Messen der Fluoreszenzeigenschaft des zweiten Aliquots zu einer Vielzahl von Zeitpunkten nach Kontakt mit dem Phosphatdonor;
das Bestimmen des Grads der Veränderung oder der Rate der Veränderung in der Fluoreszenzeigenschaft des ersten und zweiten Aliquots;
das Vergleichen des Grads oder der Rate der Veränderung in dem ersten und zweiten Aliquot;
wobei ein Unterschied im Grad oder in der Rate der Veränderung der Fluoreszenzeigenschaft in dem ersten und zweiten Aliquot anzeigt, dass der Substratkandidat eine Peptideinheit besitzt, die die Fluoreszenzeigenschaft verändert.

32. Verfahren nach Anspruch 30 oder 31, wobei die Menge bestimmt wird durch das Ins-Verhältnissetzen der Emissionsamplitude oder das Ins-Verhältnissetzen der Anregungsamplitude.

33. Verfahren nach Anspruch 25, ferner umfassend das Isolieren eines Mitglieds, das eine veränderte Fluoreszenzeigenschaft hat.

34. Verfahren nach einem der Ansprüche 23 bis 33, wobei der Schritt des Bestimmens der Veränderungsrate das Messen der Fluoreszenzeigenschaft zu einer Vielzahl von Zeitpunkten nach Induzieren der intrazellulären Proteinkinaseaktivität umfasst.

35. Verfahren nach einem der Ansprüche 23 bis 34, wobei die Wirtszelle in eine clonale Population expandiert wird.

## Revendications

1. Procédé permettant de déterminer si un échantillon contient une activité protéine kinase comprenant :
la mise en contact de l'échantillon avec un donneur de phosphate et un substrat protéique fluorescent pour une protéine kinase, le substrat protéique comprenant une protéine fluorescente présentant au moins 85 % d'identité de séquence sur 150 acides aminés contigus quelconques avec la séquence en acides aminés de SEQ ID NO : 2, contenant en outre un site de phosphorylation choisi dans le groupe constitué par RRFSV au niveau des positions 25 à 29, RRFSA au niveau des positions 69 à 73, KRDSM au niveau des positions 214 à 218, MRKGSCLF au niveau des positions 1 à 8, MRKGSLLF au niveau des positions 1 à 8, MRRESLLF au niveau des positions 1 à 8, MRRDSCLF au niveau des positions 1 à 8, MSRRDSCF au niveau des positions 1 à 8, MSKRRDLS au niveau des positions 1 à 8, MSRRRRSI au niveau des positions 1 à 8, MRRRRSII au niveau des positions 1 à 8, MRRRRSIII au niveau des positions -1 à 8, MRRRRSIIIF au niveau des positions -2 à 8 et MRRRRSIIIIF au niveau des positions -3 à 8, où le substrat protéique présente une propriété fluorescente différente à l'état phosphorylé par rapport à l'état non phosphorylé ;
l'excitation du substrat protéique ; et
la mesure de la quantité d'une propriété fluorescente qui diffère à l'état non phosphorylé et à l'état phosphorylé, moyennant quoi une quantité qui est cohérente avec la présence du substrat protéique dans son état phosphorylé indique la présence d'une activité protéine kinase.

2. Procédé de la revendication 1 permettant de déterminer la quantité d'activité d'une protéine kinase dans un échantillon, dans lequel la mesure de la quantité d'une propriété fluorescente dans l'échantillon comprend la mesure de la quantité à deux points du temps ou plus après la mise en contact de l'échantillon avec un donneur de phosphate et un substrat protéique fluorescent, et la détermination de la quantité de changement ou du taux de changement de la quantité mesurée, moyennant quoi la quantité ou le taux de changement de la quantité mesurée reflète la quantité d'activité protéine kinase dans l'échantillon.

3. Procédé *in vitro* permettant de déterminer si une cellule présente une activité protéine kinase, comprenant les étapes consistant à :
fournir une cellule hôte transfectée comprenant une molécule d'acide nucléique recombinant comprenant des séquences de contrôle de l'expression liées de manière fonctionnelle à une séquence en acide nucléique codant pour l'expression d'un substrat protéique fluorescent pour une protéine kinase, le substrat protéique comprenant une protéine fluorescente présentant au moins 85 % d'identité de séquence sur 150 acides aminés contigus quelconques avec la séquence d'acides aminés de SEQ ID NO : 2, contenant en outre un site de phosphorylation choisi dans le groupe constitué par RRFSV au niveau des positions 25 à 29, RRFSA au niveau des positions 69 à 73, KRDSM au niveau des positions 214 à 218, MRKGSCLF au niveau des positions 1 à 8, MRKGSLLF au niveau des positions 1 à 8, MRRESLLF au niveau des positions 1 à 8, MRRDSCLF au niveau des positions 1 à 8, MSRRDSCF au niveau des positions 1 à 8, MSKRRDLS au niveau des positions 1 à 8, MSRRRRSI au niveau des positions 1 à 8, MRRRRSII au niveau des positions 1 à 8, MRRRRSIII au niveau des positions -1 à 8, MRRRRSIIIF au niveau des positions -2 à 8 et MRRRRSIIIIF au niveau des positions -3 à 8, où le substrat protéique présente une propriété fluorescente différente à l'état phosphorylé par rapport à l'état non phosphorylé, la cellule exprimant le substrat protéique fluorescent ;
exciter le substrat protéique dans la cellule ; et
mesurer la quantité d'une propriété fluorescente qui diffère dans les états non phosphorylé et phosphorylé, où la présence de la propriété fluorescente associée à l'état fluorescent indique la présence d'une activité protéine kinase dans la cellule.

4. Procédé de la revendication 3, dans lequel la cellule est en outre transfectée avec un vecteur d'expression comprenant des séquences de contrôle de l'expression liées de manière fonctionnelle à une séquence d'acide nucléique codant pour l'expression de la protéine kinase.

5. Procédé de la revendication 3 ou 4, dans lequel l'étape consistant à fournir une cellule hôte transfectée comprend l'induction de l'expression du substrat protéique pour produire une augmentation soudaine de l'expression du substrat protéique et l'étape consistant à mesurer la quantité d'une propriété fluorescente comprend la mesure de la quantité à un premier et à un second temps après l'expression du substrat protéique et la détermination de la différence entre les quantités mesurées au premier et au second temps.

6. Procédé *in vitro* permettant de déterminer la quantité d'activité d'une protéine kinase dans un échantillon provenant d'un organisme, comprenant les étapes consistant à :
fournir un échantillon provenant d'un organisme ayant une cellule qui exprime un substrat protéique fluorescent pour une protéine kinase, le substrat protéique comprenant une protéine fluorescente présentant au moins 85 % d'identité de séquence sur 150 acides aminés contigus quelconques avec la séquence en acides aminés de SEQ ID NO: 2, contenant en outre un site de phosphorylation choisi dans le groupe constitué par RRFSV au niveau des positions 25 à 29, RRFSA au niveau des positions 69 à 73, KRDSM au niveau des positions 214 à 218, MRKGSCLF au niveau des positions 1 à 8, MRKGSLLF au niveau des positions 1 à 8, MRRESLLF au niveau des positions 1 à 8, MRRDSCLF au niveau des positions 1 à 8, MSRRDSCF au niveau des positions 1 à 8, MSKRRDLS au niveau des positions 1 à 8, MSRRRRSI au niveau des positions 1 à 8, MRRRRSII au niveau des positions 1 à 8, MRRRRSIII au niveau des positions -1 à 8, MRRRRSIIIF au niveau des positions -2 à 8 et MRRRRSIIIIF au niveau des positions -3 à 8, où le substrat protéique présente une propriété fluorescente différente à l'état phosphorylé par rapport à l'état non phosphorylé ;
mettre en contact l'échantillon avec un donneur de phosphate ;
exciter le substrat protéique ; et
mesurer la quantité d'une propriété fluorescente qui diffère à l'état non phosphorylé et à l'état phosphorylé, moyennant quoi une quantité qui est cohérente avec la présence du substrat protéique dans son état phosphorylé indique la présence d'une activité protéine kinase, et une quantité qui est cohérente avec la présence du substrat protéique dans son état non phosphorylé indique l'absence d'une activité protéine kinase.

7. Procédé de la revendication 6, dans lequel l'échantillon est un homogénat cellulaire.

8. Procédé permettant de déterminer si un composé modifie l'activité d'une protéine kinase comprenant les étapes consistant à :
mettre en contact un échantillon contenant une quantité connue d'activité protéine kinase avec le composé, un donneur de phosphate pour la protéine kinase et un substrat protéique fluorescent pour une protéine kinase, le substrat protéique comprenant une protéine fluorescente présentant au moins 85 % d'identité de séquence sur 150 acides aminés contigus quelconques avec la séquence en acides aminés de SEQ ID NO : 2, contenant en outre un site de phosphorylation choisi dans le groupe constitué par RRFSV au niveau des positions 25 à 29, RRFSA au niveau des positions 69 à 73, KRDSM au niveau des positions 214 à 218, MRKGSCLF au niveau des positions 1 à 8, MRKGSLLF au niveau des positions 1 à 8, MRRESLLF au niveau des positions 1 à 8, MRRDSCLF au niveau des positions 1 à 8, MSRRDSCF au niveau des positions 1 à 8, MSKRRDLS au niveau des positions 1 à 8, MSRRRRSI au niveau des positions 1 à 8, MRRRRSII au niveau des positions 1 à 8, MRRRRSIII au niveau des positions -1 à 8, MRRRRSIIIF au niveau des positions -2 à 8 et MRRRRSIIIIF au niveau des positions -3 à 8, où le substrat protéique présente une propriété fluorescente différente à l'état phosphorylé par rapport à l'état non phosphorylé ;
exciter le substrat protéique ;
mesurer la quantité d'activité protéine kinase dans l'échantillon en fonction de la quantité de changement ou du taux de changement d'une propriété fluorescente qui diffère dans les états non phosphorylé et phosphorylé ; et
comparer la quantité d'activité dans l'échantillon avec une activité étalon pour la même quantité de la protéine kinase, moyennant quoi une différence entre la quantité d'activité protéine kinase dans l'échantillon et l'activité étalon indique que le composé modifie l'activité de la protéine kinase.

9. Procédé pour déterminer si un composé modifie l'activité protéine kinase dans une cellule comprenant les étapes consistant à :
fournir une première et une seconde cellule hôte transfectée présentant une activité protéine kinase et exprimant un substrat protéique fluorescent pour une protéine kinase, le substrat protéique comprenant une protéine fluorescente présentant au moins 85 % d'identité de séquence sur 150 acides aminés contigus quelconques avec la séquence en acides aminés de SEQ ID NO : 2, contenant en outre un site de phosphorylation choisi dans le groupe constitué par RRFSV au niveau des positions 25 à 29, RRFSA au niveau des positions 69 à 73, KRDSM au niveau des positions 214 à 218, MRKGSCLF au niveau des positions 1 à 8, MRKGSLLF au niveau des positions 1 à 8, MRRESLLF au niveau des positions 1 à 8, MRRDSCLF au niveau des positions 1 à 8, MSRRDSCF au niveau des positions 1 à 8, MSKRRDLS au niveau des positions 1 à 8, MSRRRRSI au niveau des positions 1 à 8, MRRRRSII au niveau des positions 1 à 8, MRRRRSIII au niveau des positions -1 à 8, MRRRRSIIIF au niveau des positions -2 à 8 et MRRRRSIIIIF au niveau des positions -3 à 8, où le substrat protéique présente une propriété fluorescente différente à l'état phosphorylé par rapport à l'état non phosphorylé ;
mettre en contact la première cellule avec une quantité du composé ;
mettre en contact la seconde cellule avec une quantité différente du composé ;
exciter le substrat protéique dans les première et seconde cellules ;
mesurer la quantité d'activité protéine kinase dans les cellules en fonction de la quantité de changement ou du taux de changement d'une propriété fluorescente qui diffère dans les états non phosphorylé et phosphorylé dans les première et seconde cellules ; et
comparer la quantité dans les première et seconde cellules, moyennant quoi une différence de la quantité indique que le composé modifie l'activité protéine kinase dans la cellule.

10. Procédé de la revendication 9, dans lequel les cellules sont transfectées avec un vecteur d'expression comprenant des séquences de contrôle de l'expression liées de manière fonctionnelle à une séquence d'acide nucléique codant pour l'expression de la protéine kinase.

11. Procédé de l'une quelconque des revendications 1 à 10, dans lequel la propriété fluorescente est l'émission fluorescente autour du maximum d'émission du substrat à l'état phosphorylé.

12. Procédé de l'une quelconque des revendications 1 à 11, dans lequel la quantité d'activité protéine kinase est déterminée par la détermination du rapport d'amplitude d'émission ou la détermination du rapport d'amplitude d'excitation.

13. Substrat protéique fluorescent pour une protéine kinase comprenant une protéine fluorescente présentant au moins 85 % d'identité de séquence sur 150 acides aminés contigus quelconques avec la séquence en acides aminés de SEQ ID NO : 2, contenant en outre un site de phosphorylation choisi dans le groupe constitué par RRFSV au niveau des positions 25 à 29, RRFSA au niveau des positions 69 à 73, KRDSM au niveau des positions 214 à 218, MRKGSCLF au niveau des positions 1 à 8, MRKGSLLF au niveau des positions 1 à 8, MRRESLLF au niveau des positions 1 à 8, MRRDSCLF au niveau des positions 1 à 8, MSRRDSCF au niveau des positions 1 à 8, MSKRRDLS au niveau des positions 1 à 8, MSRRRRSI au niveau des positions 1 à 8, MRRRRSII au niveau des positions 1 à 8, MRRRRSIII au niveau des positions -1 à 8, MRRRRSIIIF au niveau des positions -2 à 8 et MRRRRSIIIIF au niveau des positions -3 à 8, où le substrat protéique présente une propriété fluorescente différente à l'état phosphorylé par rapport à l'état non phosphorylé.

14. Substrat protéique selon la revendication 13, où la protéine fluorescente est le variant P4, P4-3, W7, W2, S65T, P4-1, S65A, S65L, Y66F ou Y66W.

15. Substrat protéique de l'une ou l'autre des revendications 13 ou 14, dans lequel la protéine fluorescente comprend en outre une mutation affectant le repliement choisie parmi T44A, I64L, V68L, S72A, I99S, Y145I, N146I, M153 (T ou A), V163A, I167T, S175G, S205T et N212K.

16. Substrat protéique de l'une quelconque des revendications 13 à 15, comprenant en outre les substitutions S65A, N149K, V163A et I167T.

17. Substrat protéique de l'une quelconque des revendications 13 à 16, comprenant en outre la substitution H217S.

18. Substrat protéique de l'une quelconque des revendications 13 à 17, comprenant en outre la substitution E171K et/ou I172V.

19. Substrat protéique de l'une quelconque des revendications 13 à 18, où le substrat protéique est une protéine de fusion.

20. Molécule d'acide nucléique codant pour l'expression d'un substrat protéique fluorescent tel que défini dans l'une quelconque des revendications 13 à 19.

21. Molécule d'acide nucléique recombinant comprenant des séquences de contrôle de l'expression liées de manière fonctionnelle à une séquence d'acide nucléique codant pour l'expression d'un substrat protéique fluorescent pour une protéine kinase comprenant une protéine fluorescente présentant au moins 85 % d'identité de séquence sur 150 acides aminés contigus quelconques avec la séquence en acides aminés de SEQ ID NO : 2, contenant en outre un site de phosphorylation choisi dans le groupe constitué par RRFSV au niveau des positions 25 à 29, RRFSA au niveau des positions 69 à 73, KRDSM au niveau des positions 214 à 218, MRKGSCLF au niveau des positions 1 à 8, MRKGSLLF au niveau des positions 1 à 8, MRRESLLF au niveau des positions 1 à 8, MRRDSCLF au niveau des positions 1 à 8, MSRRDSCF au niveau des positions 1 à 8, MSKRRDLS au niveau des positions 1 à 8, MSRRRRSI au niveau des positions 1 à 8, MRRRRSII au niveau des positions 1 à 8, MRRRRSIII au niveau des positions -1 à 8, MRRRRSIIIF au niveau des positions -2 à 8 et MRRRRSIIIIF au niveau des positions -3 à 8, où le substrat protéique présente une propriété fluorescente différente à l'état phosphorylé par rapport à l'état non phosphorylé.

22. Cellule hôte transfectée comprenant une molécule d'acide nucléique recombinant comprenant des séquences de contrôle de l'expression liées de manière fonctionnelle à une séquence d'acide nucléique codant pour l'expression d'un substrat protéique fluorescent pour une protéine kinase comprenant une protéine fluorescente présentant au moins 85 % d'identité de séquence sur 150 acides aminés contigus quelconques avec la séquence en acides aminés de SEQ ID NO : 2, contenant en outre un site de phosphorylation choisi dans le groupe constitué par RRFSV au niveau des positions 25 à 29, RRFSA au niveau des positions 69 à 73, KRDSM au niveau des positions 214 à 218, MRKGSCLF au niveau des positions 1 à 8, MRKGSLLF au niveau des positions 1 à 8, MRRESLLF au niveau des positions 1 à 8, MRRDSCLF au niveau des positions 1 à 8, MSRRDSCF au niveau des positions 1 à 8, MSKRRDLS au niveau des positions 1 à 8, MSRRRRSI au niveau des positions 1 à 8, MRRRRSII au niveau des positions 1 à 8, MRRRRSIII au niveau des positions -1 à 8, MRRRRSIIIF au niveau des positions -2 à 8 et MRRRRSIIIIF au niveau des positions -3 à 8, où le substrat protéique présente une propriété fluorescente différente à l'état phosphorylé par rapport à l'état non phosphorylé.

23. Procédé *in vitro* de criblage d'une collection de cellules hôtes transfectées comprenant :
la fourniture d'une collection de cellules hôtes transfectées comprenant au moins 10 membres différents de cellules hôtes, chaque membre exprimant un substrat protéique fluorescent candidat différent, le substrat candidat comprenant une protéine fluorescente présentant au moins 85 % d'identité de séquence sur 150 acides aminés contigus quelconques avec la séquence en acides aminés de SEQ ID NO : 2 et un radical peptidique variable au sein d'environ vingt acides aminés de l'extrémité amino du radical protéique fluorescent, où la protéine fluorescente présente une propriété fluorescente ;
la mesure de la propriété fluorescente dans un échantillon comprenant au moins un membre de cellule hôte avant et après l'augmentation ou la diminution de l'activité protéine kinase intracellulaire dans la cellule ; et
la détermination du degré de changement ou du taux de changement de la propriété fluorescente après augmentation ou diminution de l'activité protéine kinase intracellulaire ;
moyennant quoi un changement du degré ou du taux de la propriété fluorescente indique que le substrat candidat possède un radical peptidique qui peut être phosphorylé par la protéine kinase et dont la phosphorylation modifie la propriété fluorescente.

24. Procédé de la revendication 23, dans lequel le radical peptidique variable est au sein d'environ 5 acides aminés de l'extrémité amino.

25. Procédé de la revendication 23, comprenant en outre la détermination du changement dans une pluralité de membres de cellules hôtes et la comparaison du degré de changement ou du taux de changement entres les membres des cellules hôtes ; moyennant quoi la comparaison indique dans les substrats candidats le changement relatif de la propriété fluorescente après phosphorylation.

26. Procédé de l'une quelconque des revendications 23 à 25, dans lequel la cellule hôte est cotransfectée avec un vecteur d'expression qui exprime une protéine kinase comprenant des séquences de contrôle de l'expression liées de manière fonctionnelle à une séquence qui code pour l'expression de la protéine kinase.

27. Procédé de la revendication 26, dans lequel l'étape consistant à augmenter l'activité protéine kinase intracellulaire comprend l'élévation de l'AMPc intracellulaire pour activer une protéine kinase dépendante de l'AMPc, l'élévation du GMPc pour activer une protéine kinase dépendante du GMPc, l'élévation du calcium libre cytosolique pour activer une protéine kinase dépendante du Ca2+/calmoduline des types I, II ou IV, ou l'administration de myristate acétate de phorbol pour activer la protéine kinase C.

28. Procédé de l'une quelconque des revendications 23 à 26, dans lequel l'étape consistant à diminuer l'activité protéine kinase intracellulaire comprend la culture de la cellule pendant un temps suffisant pour que la cellule perde la séquence qui code pour l'expression de la protéine kinase.

29. Procédé de l'une quelconque des revendications 23 à 28, comprenant en outre l'isolement de la molécule d'acide nucléique recombinant codant pour l'expression du substrat candidat.

30. Procédé *in vitro* de criblage d'une collection de cellules hôtes transfectées comprenant :
la fourniture d'un lysat provenant de chacun d'au moins un membre d'une collection de cellules hôtes transfectées comprenant au moins 10 membres différents de cellules hôtes, chaque membre exprimant un substrat protéique fluorescent candidat différent, le substrat candidat comprenant une protéine fluorescente présentant au moins 85 % d'identité de séquence sur 150 acides aminés contigus quelconques avec la séquence en acides aminés de SEQ ID NO : 2 et un radical peptidique variable autour de l'extrémité amino du radical protéique fluorescent, où la protéine fluorescente présente une propriété fluorescente ; la mise en contact du lysat avec un donneur de phosphate ;
la mesure de la propriété fluorescente à partir d'au moins un lysat avant et après la mise en contact du lysat avec une protéine kinase ; et
la détermination du degré de changement ou du taux de changement de la propriété fluorescente après la mise en contact du lysat avec une activité protéine kinase intracellulaire ;
moyennant quoi un changement du degré ou du taux de la propriété fluorescente indique que le substrat candidat possède un radical peptidique qui peut être phosphorylé par la protéine kinase et dont la phosphorylation modifie la propriété fluorescente.

31. Procédé de la revendication 30, comprenant la division du lysat en un premier et un second aliquots ;
la mise en contact du premier et du second aliquots avec un donneur de phosphate ;
la mise en contact du premier aliquot avec une protéine kinase, mais pas la mise en contact du second aliquot avec la protéine kinase ;
la mesure de la propriété fluorescente à partir du premier aliquot avant et après la mise en contact du lysat avec la protéine kinase ;
la mesure de la propriété fluorescente à partir de second aliquot à une pluralité de points du temps après contact avec le donneur de phosphate ;
la détermination du degré de changement ou du taux de changement de la propriété fluorescente des premier et second aliquots ;
la comparaison du degré ou du taux de changement dans les premier et second aliquots ;
moyennant quoi une différence du degré ou du taux de changement de la propriété fluorescente dans les premier et second aliquots indique que le substrat candidat possède un radical peptidique qui modifie la propriété fluorescente.

32. Procédé de la revendication 30 ou 31, dans lequel la quantité est déterminée par le rapport d'amplitude d'émission ou le rapport d'amplitude d'excitation.

33. Procédé de la revendication 25, comprenant en outre l'isolement d'un membre ayant une propriété fluorescente modifiée.

34. Procédé de l'une quelconque des revendications 23 à 33, dans lequel l'étape consistant à déterminer le taux de changement comprend la mesure de la propriété fluorescente à une pluralité de points du temps après l'induction de l'activité protéine kinase intracellulaire.

35. Procédé de l'une quelconque des revendications 23 à 34, dans lequel la cellule hôte est expansée en une population clonale.
